# EUROPEAN PATENT SPECIFICATION

(11) **EP 3 071 284 B1**
(45) Date of publication and mention of the grant of the patent: **14.08.2019**
(21) Application number: 14864273.9
(22) Date of filing: 21.11.2014
(51) Int. Cl.: A61M 25/14, A61M 25/00, A61M 1/36

(54) **CATHETER ASSEMBLY INCLUDING A MULTI-LUMEN CONFIGURATION**
KATHETERANORDNUNG MIT EINER MEHRLUMIGEN KONFIGURATION
ENSEMBLE CATHÉTER DOTÉ D'UNE CONFIGURATION À PLUSIEURS LUMIÈRES

(30) Priority: 21.11.2013 US 201361907344 P
(43) Date of publication of application: 28.09.2016
(73) Proprietor: C.R. Bard, Inc., Murray Hill, NJ 07974 (US)
(72) Inventor: OBORN, Kelli, D., Erda, UT 84074 (US); MOEHLE, Ryan, T., Salt Lake City, UT 84117 (US); BARRON, Randy, Riverton, UT 84065 (US); CROOK, Christian, W., West Jordan, UT 84081 (US)
(74) Representative: Hoffmann Eitle
(86) International application number: PCT/US2014/066811
(87) International publication number: WO 2015/077560

(56) References cited:
- EP-A2- 2 305 337
- US-A1- 2008 082 080
- US-A1- 2009 157 051
- US-A1- 2009 187 141
- US-A1- 2010 081 986
- US-A1- 2010 081 986
- US-A1- 2012 089 070
- US-A1- 2012 089 070
- US-A1- 2013 079 752
- US-A1- 2013 261 605

## Description

### CROSS-REFERENCE TO RELATED APPLICATIONS

This application is a continuation-in-part of U.S. Patent Application No. 13/329,156, filed December 16, 2011, and titled "Catheter Assembly Including Triple-Lumen Tip," now U.S. Patent No. 8,894,601, which is a continuation of U.S. Patent Application No. 12/262,820, filed October 31, 2008, and titled "Catheter Assembly Including Triple-Lumen Tip," now U.S. Patent No. 8,092,415, which claims the benefit of U.S. Provisional Application No. 60/984,661, filed November 1, 2007, and titled "Catheter Assembly Including Triple Lumen Tip." This application also claims the benefit of U.S. Provisional Application No. 61/907,344, filed November 21, 2013, and titled "Catheter Including a Multi-Lumen Configuration."

US 2012/0089070 A1 discloses a catheter assembly comprising:an elongate catheter tube defining first, second, and third lumens, the catheter tube including a first lateral opening in fluid communication with the first lumen and a second lateral opening in fluid communication with the second lumen.

### BRIEF SUMMARY

The present invention is directed to the catheter assembly of claim 1. The dependent claims refer to preferred embodiments. The invention is defined by the appended claims. Subject matter referred as embodiments and/or inventions which are not claimed are not part of the invention.

Briefly summarized, embodiments are directed to a catheter assembly for use in accessing a vasculature or other vessel of a patient during renal replacement or other suitable therapies. In one embodiment, the catheter assembly includes a catheter body that defines at least first and second lumens. The catheter body defines a distal tip region that includes at least one venous lateral opening that is in fluid communication with the first lumen and includes a distal-facing portion, and at least one arterial lateral opening that is in fluid communication with the second lumen and includes a distal-facing portion. The at least one arterial lateral opening is opposingly positioned in a substantially unstaggered configuration with respect to the at least one venous lateral opening. A distal end opening is defined on the distal tip region and is sized to pass a fluid therethrough. In one embodiment, the distal end opening is in fluid communication with a third lumen of the catheter body that can withstand high fluid flow rates associated with power injection of contrast media, for instance.

In another embodiment, a catheter assembly including a catheter body defining a first lumen and a second lumen is disclosed. The catheter body includes a distal tip region, which in turn includes a nose portion that defines a distally converging outer surface. A venous lateral opening, in fluid communication with the first lumen, is partially defined on the distally converging outer diameter. An arterial lateral opening, in fluid communication with the second lumen, is also partially defined on the distally converging outer diameter. The venous and arterial lateral openings are symmetrically disposed in a substantially unstaggered position with respect to one another. The distal tip portion further includes a distal end opening in fluid communication with one of the venous and arterial lumens and is sized to pass a guidewire therethrough.

According to the invention, the first and second lumens each generally include a reniform cross sectional shape, while the third lumen is substantially round, and may be interposed between the first and second lumens, and may be power injectable.

These and other features of the present invention will become more fully apparent from the following description and appended claims, or may be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof that are illustrated in the appended drawings. It is appreciated that these drawings depict only typical embodiments of the invention and are therefore not to be considered limiting of its scope. The invention will be described and explained with additional specificity and detail through the use of the accompanying drawings in which:
FIG. 1 is a perspective view of a catheter assembly incorporating various features of an embodiment of the present invention;
FIG. 1A is a perspective view of another example of a catheter assembly configured according to one embodiment;
FIG. 2 is a perspective view of a distal tip region of the catheter assembly shown in FIG. 1, configured according to one embodiment;
FIG. 3 is a side view of the catheter distal tip region of FIG. 2;
FIG. 4 is a top view of the catheter distal tip region of FIG. 2;
FIG. 5 is an end view of the catheter distal tip region of FIG. 2;
FIG. 6 is a perspective view of the catheter distal tip region of FIG. 2, depicting various details of lateral openings defined therein;
FIG. 7A is a cross sectional view of the catheter assembly and distal tip region of FIG. 2, showing the flow of blood therethrough in a "forward" flow configuration;
FIG. 7B is a cross sectional view of the catheter assembly and distal tip region of FIG. 2, showing the flow of blood therethrough in a "reverse" flow configuration;
FIG. 8A is a cross sectional view of the catheter assembly, taken along the line 8A-8A in FIG. 4;
FIG. 8B is another cross sectional view of the catheter tip, taken along the line 8B-8B in FIG. 4;
FIG. 8C is yet another cross sectional view of the catheter tip, taken along the line 8C-8C in FIG. 4;
FIG. 8D is yet another cross sectional view of a distal tip region of the catheter assembly showing positioning of a third lumen thereof in accordance with one embodiment;
FIGS. 9A-9F depict various views of a catheter assembly including a distal tip region configured in accordance with one embodiment;
FIGS. 10A-10D are perspective, front, side, and top views, respectively, of a catheter including a distal tip region configured in accordance with one embodiment;
FIGS. 11A-11D are perspective, front, side, and top views, respectively, of a catheter including a distal tip region configured in accordance with one embodiment;
FIGS. 12A-12D are perspective, front, side, and top views, respectively, of a catheter including a distal tip region configured in accordance with one embodiment;
FIGS. 13A-13D are perspective, front, side, and top views, respectively, of a catheter including a distal tip region configured in accordance with one embodiment;
FIGS. 14A-14D are perspective, front, side, and top views, respectively, of a catheter including a distal tip region configured in accordance with one embodiment;
FIGS. 15A-15D are perspective, front, side, and top views, respectively, of a catheter including a distal tip region configured in accordance with one embodiment;
FIGS. 16A-16D are perspective, front, side, and top views, respectively, of a catheter including a distal tip region configured in accordance with one embodiment;
FIGS. 17A-17D are perspective, front, side, and top views, respectively, of a catheter including a distal tip region configured in accordance with one embodiment;
FIGS. 18A-18D are perspective, front, side, and top views, respectively, of a catheter including a distal tip region configured in accordance with one embodiment;
FIGS. 19A-19D are perspective, front, side, and top views, respectively, of a catheter including a distal tip region configured in accordance with one embodiment;
FIGS. 20A-20D are perspective, front, side, and top views, respectively, of a catheter including a distal tip region configured in accordance with one embodiment;
FIG. 21 is a perspective view of a catheter assembly according to one embodiment;
FIGS. 22A and 22B are various perspective views of a distal portion of the catheter assembly of FIG. 21;
FIGS. 23A-23C are various cross-sectional views of the distal portion of the catheter assembly of FIG. 21;
FIG. 24 is a cross-sectional view of the catheter assembly of FIG. 21;
FIG. 25 is a cross-sectional view of a catheter assembly according to one embodiment;
FIG. 26 is a cross-sectional view of a catheter assembly according to one embodiment;
FIG. 27 is a perspective view of a catheter assembly according to one embodiment; and
FIG. 28 is a cross-sectional view of the catheter assembly of FIG. 27.

### DETAILED DESCRIPTION OF SELECTED EMBODIMENTS

Reference will now be made to figures wherein like structures will be provided with like reference designations. It is understood that the drawings are diagrammatic and schematic representations of example embodiments, and are not limiting of the embodiments nor are they necessarily drawn to scale.

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

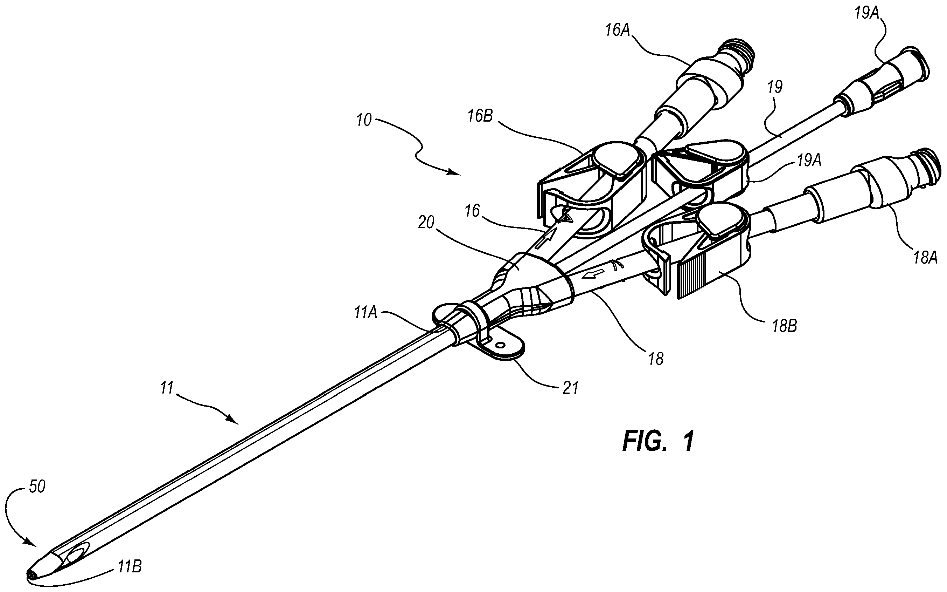

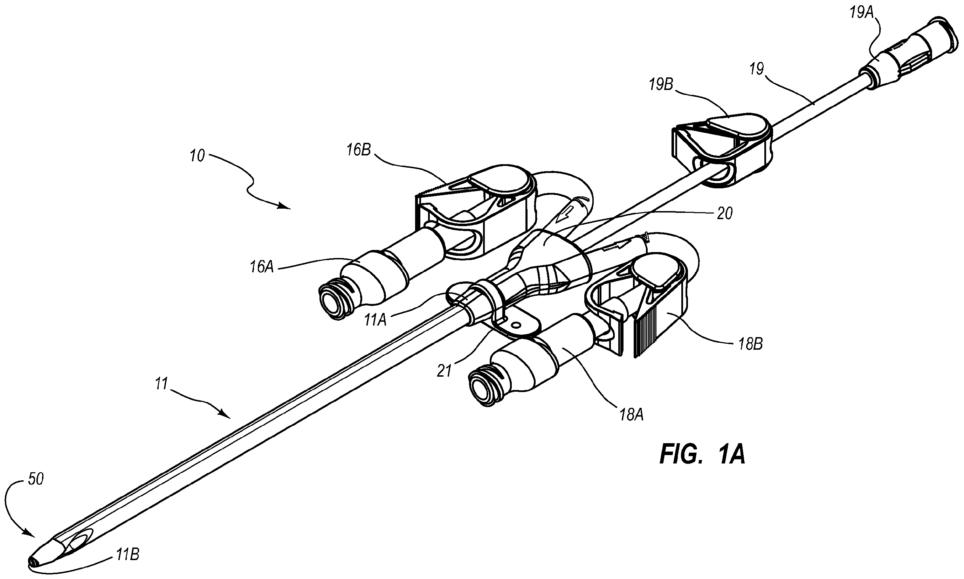

FIGS. 1-20D depict various features of embodiments of the present invention, which are generally directed to an acute catheter assembly for use in accessing a vasculature or other vessel of a patient during renal replacement therapies such as hemodialysis or blood purification, though the principles of the present invention may be extended to other catheters employed in other uses in addition to these. Such acute catheters are typically employed in short-term placement scenarios such as a placement of less than 30 days, though the principles to be described herein can also apply to mid-term and long term catheter placements as well.

In accordance with one example embodiment, the catheter assembly includes a distal tip region defining separate venous and arterial lateral openings, in fluid communication with corresponding venous and arterial lumens that are employed for simultaneously infusing and aspirating blood from a vein or other vessel of a patient's vasculature during hemodialysis treatments. The venous and arterial lateral openings are disposed in a substantially equivalent, non-staggered position with respect to one another so as to enable positioning thereof in a predetermined region of the vasculature. This notwithstanding, the lateral openings are configured to reduce the likelihood of recirculation by the arterial segment of treated blood just returned to the vessel by the venous segment, thus increasing catheter efficiency. Moreover, the lateral openings can be operated in a reverse flow configuration without significantly impacting catheter efficiency during hemodialysis.

Embodiments of the catheter assembly to be described herein further include a distal end opening in fluid communication with a lumen of the catheter configured to withstand relatively high pressure and flow rates typically associated with power injection. This enables aspiration or infusion of fluids to occur via this lumen independently of the venous and arterial lumens. "Power injection" is defined herein to include fluid infusion under relatively high flow rates and/or relatively high pressures. For instance, in one embodiment power injection includes fluid infusion through a catheter lumen at a flow rate of between about three and about eight milliliters per second, and/or at a pressure of between about 0.344 MPa and about 1.723 MPa (50 and about 250 psi).

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Further, the words "including," "has," and "having," as used herein, including the claims, shall have the same meaning as the word "comprising."

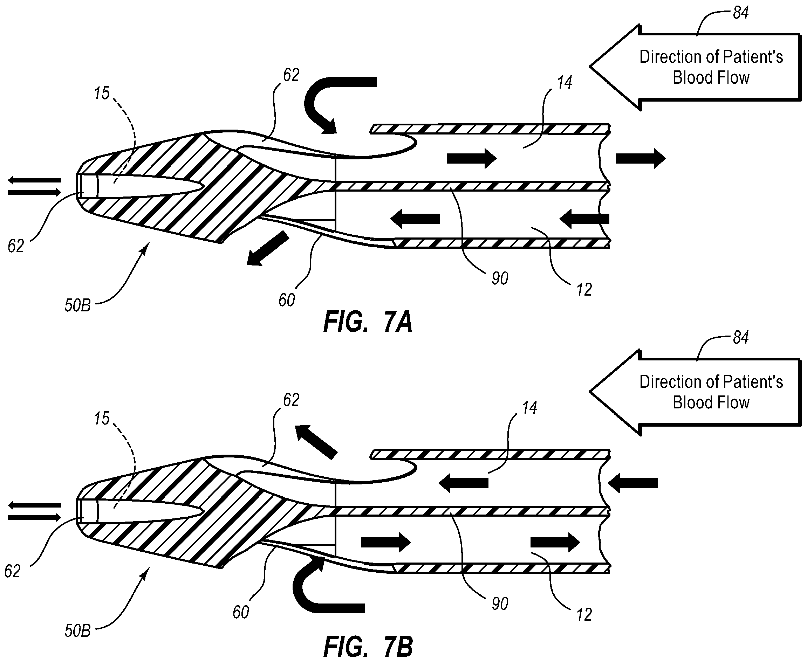

Reference is first made to FIG. 1, which depicts various features of a hemodialysis catheter assembly, generally designated at 10, according to one example embodiment. As shown, the catheter 10 includes an elongate catheter body 11 including a proximal end 11A and a distal end 11B. The elongate catheter body 11 defines a first lumen 12, a second lumen 14, and a third lumen 15 (FIG. 7A) that longitudinally extend from the proximal end 11A to the distal end 11B thereof. The lumens 12, 14, and 15 can have one or more cross sectional shapes along their respective lengths, including round, oval, D-cross sectional shapes, or any combination thereof. In one embodiment, the first and second lumens 12, 14 are sized so as to accommodate fluid flow rates required for hemodialysis, *i.e.,* about 300 milliliters/min. at about 0.033 MPa (250 mm Hg) pressure. In one embodiment, the third lumen is sized with a diameter of about 0.889 mm to about 0.965 mm (0.035 to about 0.038 inch) to accommodate blood draws and fluid aspiration/infusion therethrough.

A trifurcating hub 20 is included at the catheter body proximal end 11A, providing fluid communication between the first, second, and third lumens 12, 14, 15 and arterial extension leg 16, venous extension leg 18, and power extension leg 19, respectively. The extension legs 16, 18, 19 each include a luer connector 16A, 18A, 19A, and a clamp 16B, 18B, 19B. So configured, the extension legs 16, 18 provide fluid communication with the first and second lumens 12 and 14 so as to enable the infusion or aspiration of fluids from the central venous system of a patient. As such, fluid infusion or aspiration devices, such as a hemodialysis apparatus for example, may be connected to the catheter assembly 10 via the luer connectors 16A, 18A, thus providing intravascular access to the patient. Similarly, the extension leg 19 provides fluid communication with the third lumen 15 to enable fluid infusion/aspiration from the vein when a corresponding device is connected thereto via the connector 19A. Note that the respective positions and configurations of the extension legs detailed here can change according to a particular catheter assembly design and therefore not be viewed as limiting. The catheter body 11 further includes a suture wing 21 for providing securement of the catheter body to the patient.

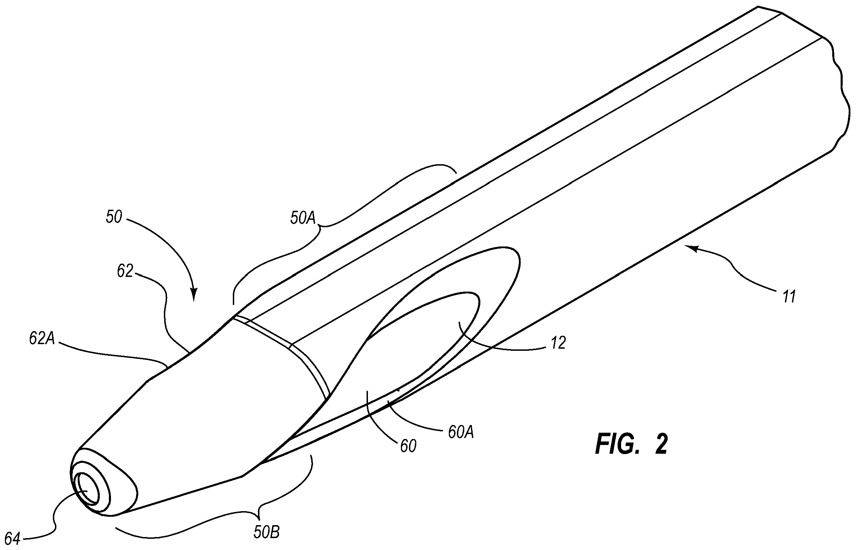

FIG. 2 shows the catheter assembly 10 according to another example embodiment, wherein the extension legs 16, 18 each include a pre-curved portion 16C, 18C. The pre-curved portions 16C, 18C enable the extension legs 16, 18 of the catheter assembly 10 to extend downward against the patient's body once the distal portion of the catheter assembly has been placed in the vasculature to provide patient comfort.

In greater detail, the power extension leg 19 of FIGS. 1 and 2 fluidly connects to the third lumen 15 via the trifurcating hub 20. In particular, the power extension leg 19 is configured in one embodiment to enable rapid infusion, *i.e.,* power injection, of contrast media, useful for contrast-enhanced CT scan imaging, or other fluids into the patient vessel via the third lumen 15. Specifically, in one embodiment, the power extension leg 19 and third lumen 15 are configured to infuse fluids at a rate of between about 3 milliliters and about 8 milliliters per second and at a fluid pressure of between about 0.344 MPa and 1.723 MPa (50 psi and 250 psi), though other flow rates and fluid pressures may also be possible. The power extension leg 19 and third lumen 15 can also be used to remove blood or other fluids alone or during simultaneous use of the first and second lumens 12 and 14, and to monitor central venous pressure with the assistance of a transducer. The power extension leg 19 and third lumen 15 are also sufficiently sized to receive a guidewire therethrough to enable insertion of the catheter assembly over the guidewire. Note that the components of the power extension leg 19 are colored purple in one embodiment to indicate power injectability. Other colors could also be used.

Both FIGS. 1 and 2 further include a distal tip region, generally designated at 50, that is configured in accordance one example embodiment of the present invention, the details of which are given below. It should be appreciated that the distal tip region to be described below can be included with hemodialysis catheters, such as those shown in FIGS. 1 and 2, or with other catheters, such as central venous catheters, for example. Indeed, the catheter assembly according to embodiments of the present invention can be adapted for use in other applications, such as chronic dialysis treatment, or where access is desired to be gained to a vessel, such as the internal jugular, subclavian, or femoral vessels, or other body lumen of a patient. Examples of such other applications include apheresis, hemoperfusion, etc.

Reference is now made to FIGS. 2-6, which show various views of a distal tip region, generally designated at 50, of the catheter assembly 10 and configured according to one example embodiment. In detail, the distal tip region 50 generally includes a terminal catheter portion 50A and a nose portion 50B disposed distally of the terminal catheter portion to define a distal end of the catheter assembly 10. The terminal catheter portion 50A, as part of the more proximal portion of the catheter body 11, is composed of suitable material(s) that exhibit qualities, such as suitable softness to allow for ease of insertion without causing vessel trauma, and biocompatibility for enabling the catheter to operate as intended. In one embodiment, the catheter body 11 is composed of material(s) including a thermoplastic polyurethane-based resin material, specifically a polyether-based, aliphatic thermoplastic polyurethane sold under the trademark TECOFLEX, namely TECOFLEX EG-60D-B20, having a Shore D hardness of approximately 60, where "B20" refers to the radiopacifier loading, *i.e.,* barium sulfate loading at 20%. Other suitable materials can also be employed.

In contrast, the nose portion 50B includes a material relatively softer than that of the terminal catheter portion 50A so as to prevent the tip portion from damaging the vessel or other vasculature during vessel entry or transit. In one embodiment, the nose portion 50B is composed of material(s) including TECOFLEX EG-85A-B20 having a Shore A hardness of approximately 85. Notwithstanding the above description, it should be appreciated that the terminal catheter portion and the nose portion can include other materials having the desired properties as described herein and as appreciated by one skilled in the art. One non-limiting example of material that can be used for the terminal catheter portion and nose portion is silicone.

Note that in the illustrated embodiment, the nose portion 50B is joined to the terminal catheter portion 50A via a molding process during manufacture of the catheter assembly 10. In other embodiments, however, other processes for joining the nose portion to the catheter body can be employed, including for instance RF fusion (RF tipping), bonding via adhesive, integrally forming the nose portion with the catheter body, etc.

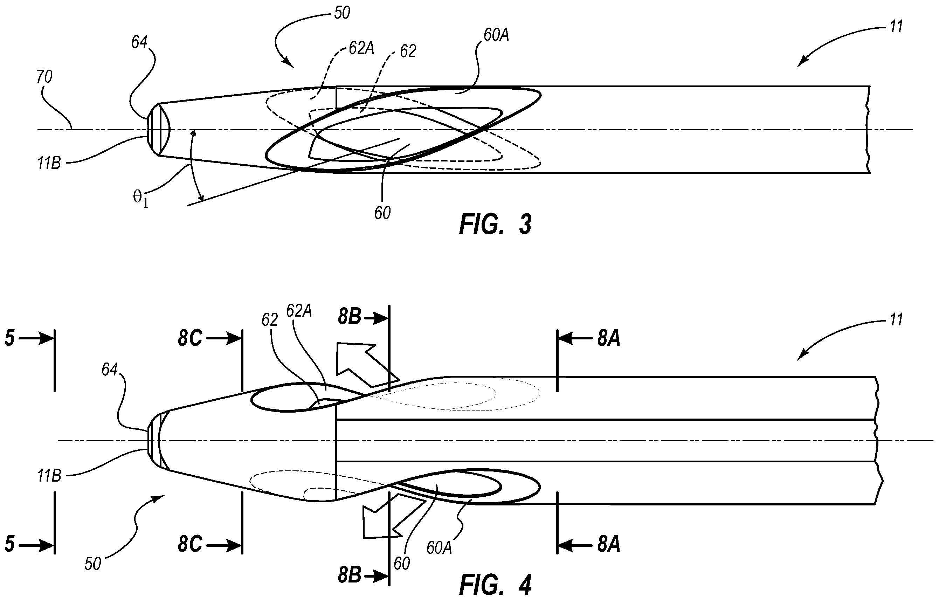

As best seen in FIGS. 3 and 4, the nose portion 50B is distally converging. In the present embodiment, the nose portion 50B is tapered so as to ease entry and passage of a distal portion of the catheter body 11 into the vasculature or other internal cavity of a patient. The nose portion 50B may be colored differently from the remainder of the catheter body 11 to indicate that the catheter assembly 10 can be employed for relatively rapid fluid aspiration and infusion via the third lumen 15 and corresponding power extension leg 19, as was described further above.

The distal tip region 50 includes various openings for enabling the infusion and aspiration of fluids while the catheter assembly 10 is placed for use within the patient vasculature. Specifically, and in accordance with one embodiment, the distal tip region includes a venous lateral opening 60, an arterial lateral opening 62, and a distal end opening 64.

In greater detail, the venous and arterial lateral openings 60 and 62 are positioned opposite one another proximate the catheter body distal end 11B and are defined in a lateral portion of an outer wall of the catheter body 11 so as to be in fluid communication with first lumen 12 and the second lumen 14, respectively, thus enabling blood or other fluids to flow via the openings to/from the lumens when the catheter assembly 10 is positioned within the patient's vasculature. The venous and arterial lateral openings 60 and 62 are defined by perimeters 60A and 62A, respectively, as best seen in FIG. 4 and described further below.

Note that each of the lateral openings 60 and 62 distally extends from the terminal catheter portion 50A into the nose portion 50B. Of course, the exact placement of the lateral openings 60 and 62 along the longitudinal length of the catheter body 11 can vary according the needs of a particular application.

FIG. 4 shows that in the present embodiment the venous and arterial lateral openings 60 and 62 are substantially un-staggered, *i.e.,* equally placed with respect to one another along the longitudinal length of the catheter body 11 such that each is substantially disposed an equal distance from the distal catheter end 11B. Such un-staggered disposal of the lateral openings 60 and 62 enables both openings to be placed proximate a desired location within the vasculature and ensures that the recirculation rate of already treated blood through the catheter assembly 10 is held relatively constant regardless the respective directions of blood travel in/out of the lateral openings. This feature is useful should reversal of blood flow directions through the catheter be necessary. In one embodiment, the recirculation rate in either direction is less than or equal to about five percent. In another embodiment, the venous and lateral openings can be staggered.

FIGS. 2-6 further show the manner in which the venous and lateral openings 60 and 62 are defined in the distal tip region 50. The lateral openings 60 and 62 can take various shapes and configurations as will be shown further below, but in the present embodiment the lateral openings are defined by angled cross-drilled cuts through the outer wall of the catheter body 11 to establish communication with the respective first or second lumens 12, 14. In one embodiment, such cuts are referred to as "skive" cuts.

In one embodiment, a long axis of each cross-drilled cut of the lateral openings 60, 62 defines in one embodiment an angle θ₁ of about 35 degrees with a longitudinal axis of the catheter body 11, though this angle can vary in one embodiment from about greater than zero to about 90 degrees. This angular character imparts both a lateral and distal directional component to fluid flow out of either lateral opening 60, 62, as represented by the flow arrows in FIG. 4, which assists in enabling low-recirculation fluid flow out of or into either lateral opening. Each lateral opening 60 and 62 in the present embodiment is defined by identical cross cuts having the same angle θ₁ with respect to the longitudinal axis 70, though it is also possible to vary the angle generally, or to vary the angle differently for each opening.

In one embodiment, the lateral openings can be defined by a compound-angle cross cut, wherein the long axis of each lateral opening defines an angle with the catheter body longitudinal axis and with a plane dividing the first lumen and the second lumen, *i.e.,* coplanar with the septum separating the first and second lumens proximal of the distal tip region.

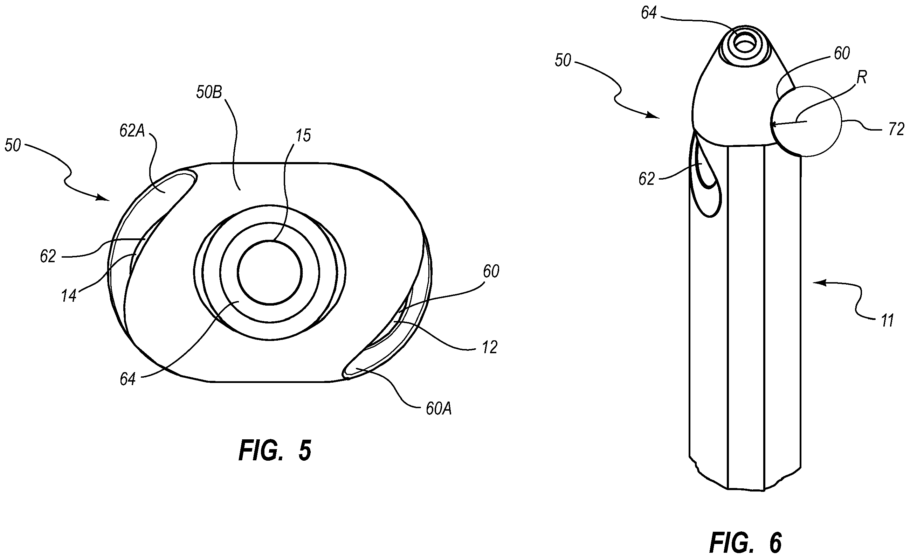

An end view of the cross cut, depicted in FIG. 6, shows that the cross cut of each opening 60 and 62 in the illustrated embodiment is made so as to generally define a semicircular cavity through a peripheral portion of the distal tip region 50. This cavity is defined by a portion of a circle 72 having a radius "R," shown in FIG. 6. In the present embodiment, the cross cut that defines the lateral openings 60 or 62 is achieved via use of a cylindrical drill bit or coring tool having a radius equal to the radius R of the circle 72 and cutting through the distal tip region 50 set at the angle θ₁. For instance, in one embodiment a drill bit having a radius of 1.587 mm (1/16 inch) is used to diagonally cross cut the venous and arterial lateral openings 60 and 62 through a catheter body defining an oblong cross section, wherein the average of the major and minor diameters is approximately 4.394 mm (0.173 inches). Note that the catheter body size in one embodiment can vary from 7-16 Fr., though other French sizes are also possible. Though shown in connection with the venous lateral opening 60, the above description applies to the arterial opening 62 as well. Note here that, though identically sized and shaped in the present embodiment, the first and second openings could have respectively differing dimensions if desired or needed for a particular application.

As a result of defining the cross cuts as just described, the venous and arterial openings 60 and 62 are defined by their respective perimeters 60A and 62A discussed above. The angle at which the cross cuts are made, together with the shape of the catheter body 11 at the point of the cuts, results in the perimeters 60A and 62A shaped as seen in the accompanying figures. As best seen in FIG. 4, each perimeter 60A and 62A defines in the present embodiment a figure-eight shape, or analemma, when viewed in a two-dimensional perspective and an elongate saddle shape when viewed in a three-dimensional perspective. Further, because a distal portion of each opening 60 and 62 is defined on a portion of the tapered nose portion 50B (best seen in FIGS. 4 and 5), each opening has a distal-facing component, best seen in FIG. 5, wherein a portion each lateral opening is distally visible.

The configuration of the venous and arterial lateral openings 60 and 62 described above provides various aspects for the catheter assembly 10. First, because of their saddle shapes, the lateral openings 60 and 62 partially extend circumferentially about the outer perimeter of the catheter body 11. This helps to prevent undesired suctioning of the distal tip region 50 to the vessel wall when one of the openings is removing blood from the vessel as the negative flow pressure of the opening is distributed about a portion of the catheter body circumference. If vessel suck-up does occur, the lateral openings 60, 62 are shaped so as to nonetheless provide acceptable fluid flow in and out of the catheter assembly 10. The relatively large size of the lateral openings 60 and 62 also assists in the prevention of occlusion or sheath formation and provides a fanned-out or wide distribution of fluid flowing out therefrom. Recirculation efficiency rates are improved as a result.

Second, the distal-facing aspect of each lateral opening 60 and 62 assists in imparting a distal direction to fluids being ejected therefrom. This enables the ejected fluid to distally flow away from one respective lateral opening and distal-to proximal flow into the other lateral opening even when the catheter body 11 is positioned against a vessel wall. In addition, the lateral openings 60, 62 are symmetrically opposed, in direction from one another, *i.e.,* a 180-degree separation as best shown in FIG. 4, so as to ensure fluid entry and exit from the lateral openings occurs on opposite sides of catheter assembly 10, further reducing recirculation. Furthermore, this symmetric positioning produces a "criss-cross" relationship between the lateral openings 60 and 62, as best seen in FIG. 3, which assists in reducing recirculation. Moreover, similar fluid flow characteristics are realized even when fluid flow through the catheter assembly 10 is reversed, as discussed further below. In addition, the lateral opening configuration described herein minimizes radical redirection of the fluid upon exiting the catheter body 11 via either of the lateral openings 60 or 62, which in turn prevents fluid turbulence and possible clotting or hemolysis.

As shown in FIGS. 2-6, the distal end opening 64 is distally located at the distal end of the distal tip region nose portion 50 and is in fluid communication with the third lumen 15 so as to enable high flow rate infusion, *i.e.,* power injection of contrast media or other fluids such as TPN nutritional fluid and medications into the vessel, as well as the removal of blood from the vessel during catheter use. In the case of infusion of contrast media or medications into the vessel, placement of the distal end opening 64 distally of the first and second openings 60 and 62 advantageously results in minimization of contrast media/medication intake into either of the first or second openings if the infusion takes place simultaneously with fluid passage through the venous and arterial openings 60 and 62, such as during hemodialysis or other treatments.

Note that, in one embodiment a guidewire can be inserted through the distal end opening 64, the third lumen 15, and the power extension leg 19 during initial or exchange catheter placement in the patient vasculature. Also note that the relatively proximate placement of the three openings 60, 62, and 64 in the distal portion of the catheter body 11 enables each opening to be placed near desired location within the vasculature, such as the superior vena cava ("SVC").

Reference is now made to FIGS. 7A and 7B in describing flow characteristics with respect to the configuration of the distal tip region 50 of the catheter assembly 10 according to the present embodiment. FIGS. 7A and 7B show the distal tip region 50 after the catheter assembly 10 has properly positioned within a vessel of a patient. Arrow 84 shows the direction of blood flow past the distal tip region 50 within the patient's vessel.

In greater detail, FIG. 7A shows fluid flow through the distal tip region 50 in a "forward" direction, wherein blood is aspirated by the second lumen 14, or "uptake" lumen, for removal from the body and treatment by a hemodialysis apparatus or for some other suitable purpose. Aspirated blood enters the second lumen 14 via the arterial lateral opening 62 of the distal tip region 50. Similarly, blood is infused, or returned, to the vessel by the first lumen 12, or "return" lumen, after treatment by a hemodialysis apparatus or some other suitable purpose. Infused blood exits the first lumen 12 from the venous lateral opening 60. Note that the lateral orientation of the venous and arterial lateral openings 60, 62 provides for low recirculation of already-treated blood within the vessel, recirculation being defined as already-treated blood that is returned to the bloodstream via the venous lumen being immediately aspirated by the arterial lumen to be re-treated. Such recirculation is undesirable as it results in lower treatment efficiency, resulting in longer treatment time.

During hemodialysis procedures, it is sometimes necessary to reverse the blood flow through the catheter assembly 10. FIG. 7B shows fluid flow through the distal tip region 50 during such a "reverse" flow situation. In contrast to the forward flow conditions of FIG. 7A, the second lumen 14 in FIG. 7B is employed to infuse blood into the vessel while the first lumen 12 aspirates blood from the vessel. In this configuration, the infused blood enters the vessel via the arterial lateral opening 62, while the aspirated blood is removed via the venous lateral opening 60. Again, the lateral orientation of the venous and arterial lateral openings 60, 62 provides for low recirculation of already-treated blood within the vessel. Thus, it is seen that low recirculation results regardless of the direction in which the catheter is operating.

FIGS. 7A and 7B further show that fluid can be aspirated or infused via the distal end opening 64 in fluid communication with the third lumen 15 before, after, or during infusion/aspiration by the venous and arterial lateral openings 60, 62. As mentioned, the third lumen 15 and distal end opening 64 are configured so as to withstand relatively high pressurized fluid flow infusion into the vessel. It is appreciated that in other embodiments, more than one of the catheter lumens can be configured for high pressurized fluid flow infusion, if desired.

It should be appreciated that the labels "venous" and "arterial" as used above in describing the various components of the present catheter assembly are employed for sake of convenience in describing aspects of present embodiments. Indeed and as just described, though the arterial lateral opening is normally employed in hemodialysis procedures for aspirating blood from the blood vessel in which the catheter is disposed and the venous lateral opening for returning already treated blood to the vessel, this can be reversed such that blood is returned via the arterial lateral opening and aspirated by the venous lateral opening. As such, embodiments of the present invention should not be considered limited by the use of this and other descriptive terminology herein.

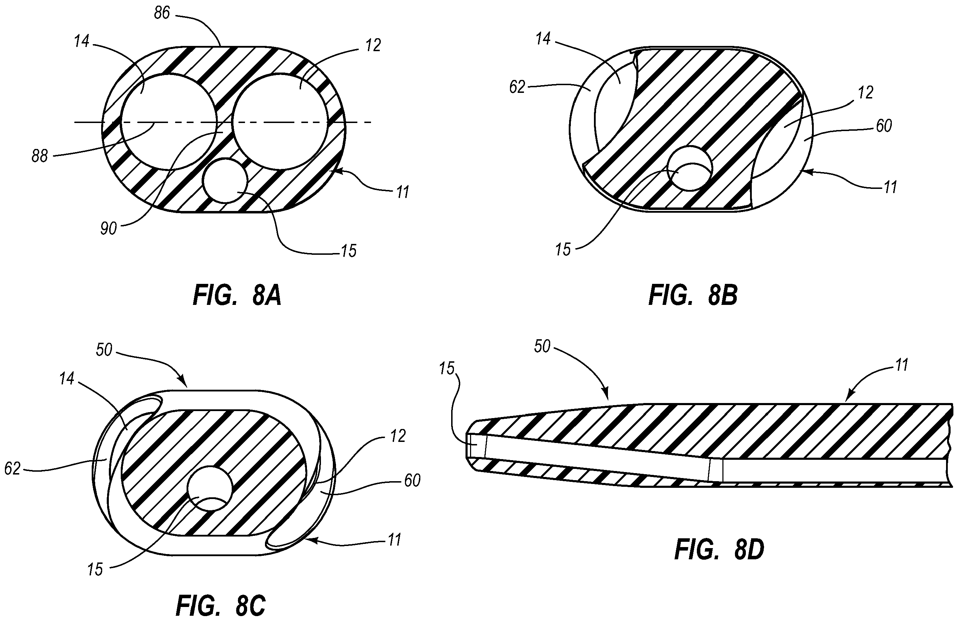

Reference is now made to FIGS. 8A-8C, which depict various details regarding the catheter body 11. In detail, FIG. 8A shows a cross sectional view of the catheter body 11 at a point proximal to the distal tip region 50, showing the first lumen 12, the second lumen 14, and the third lumen 15. The three lumens 12, 14, 15 are defined along the longitudinal length of the catheter body 11 and bounded by an outer perimeter or wall 86. The outer wall 86 of the catheter body 11 in the present embodiment defines an oblong shape and includes a transverse axis 88 that intersects the first and second lumens 12, 14 and spans the width of the catheter body. Placement of the first and second lumens 12, 14 adjacent one another, with the third lumen 15 positioned therebelow, provides a robust lumen configuration that resists inadvertent closure of lumens via kinking of the catheter body 11. In addition, the oblong cross sectional configuration of the catheter body 11 enables circular cross sectional shapes to be employed for the lumens 12, 14, and 15, which are relatively more efficient than "D"-shaped or other shaped lumens in terms of fluid flow.

As seen in FIG. 8B and as previously described, the venous lateral opening 60 is defined so that it intercepts the first lumen 12, while the arterial lateral opening is defined so that it intercepts the second lumen 14. As such, the first lumen 12 establishes fluid communication between the venous extension leg 18 and the venous lateral opening 60, while the second lumen 14 establishes fluid communication between the arterial extension leg 16 and the arterial lateral opening 62. In one embodiment, the angled cross cuts that define the venous and arterial openings 60 and 62 are made tangentially with respect to a septum 90 separating the first and second lumens 12, 14 such that the septum wall remains intact as a barrier between the two lumens.

FIGS. 8A-8C successively depict the manner in which the third lumen is raised from a bottom-central location along the length of the catheter body 11 to a central position upon its exit at the distal end opening 64, as shown in FIG. 5. Of course, other lumen position configurations are also possible.

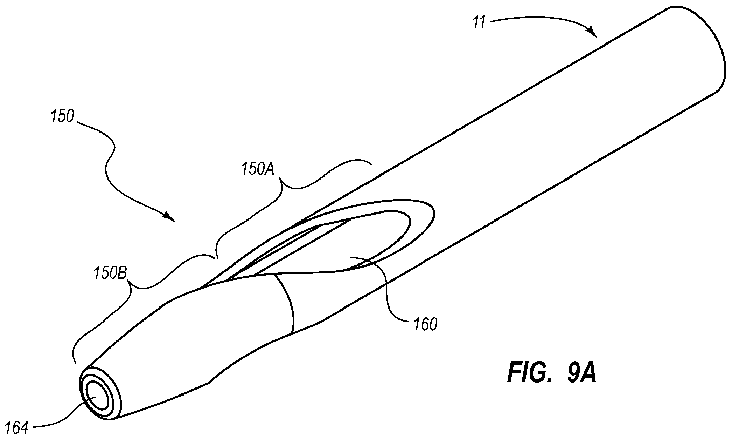

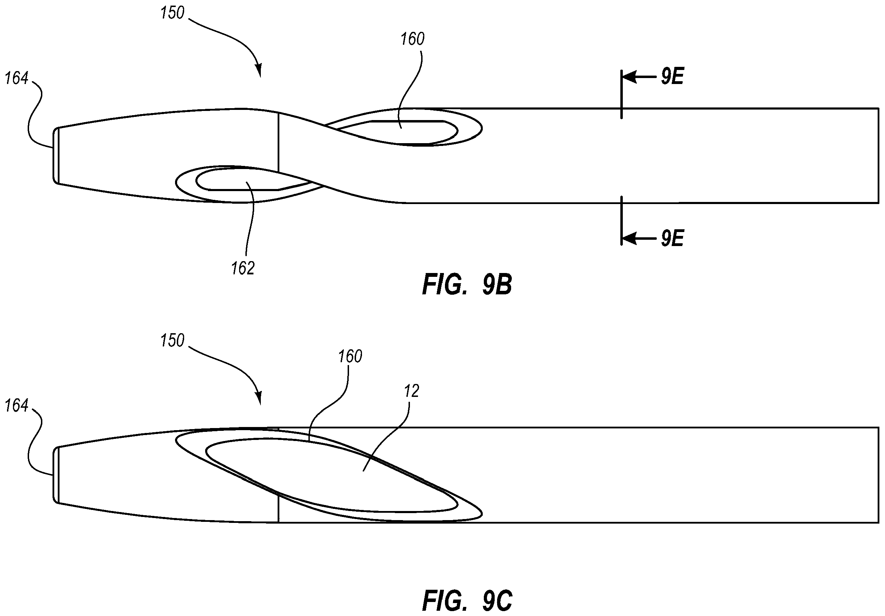

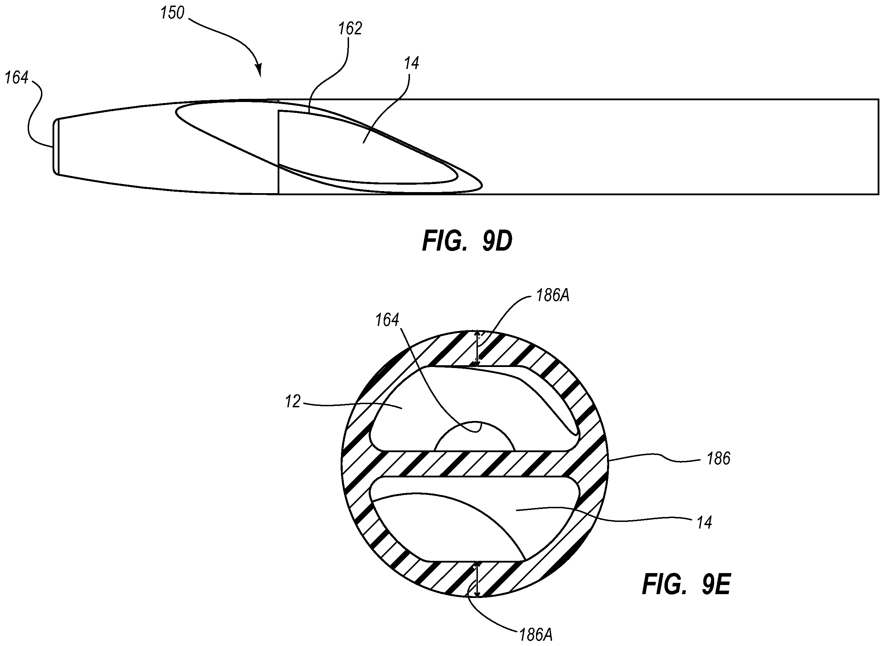

It is appreciated that various modifications may be made to the catheter assembly configurations described above. It is noted that for purposes of clarity, only selected differences between the foregoing and following embodiments are described. For instance, FIGS. 9A-9F depict a distal tip region 150 including a terminal catheter portion 150A integrally formed with the catheter body 11 and a nose portion 150B including a relatively low hardness, e.g., soft, material and joined to the terminal catheter portion 150A in a manner similar to that already described above in connection with FIGS. 2-6.

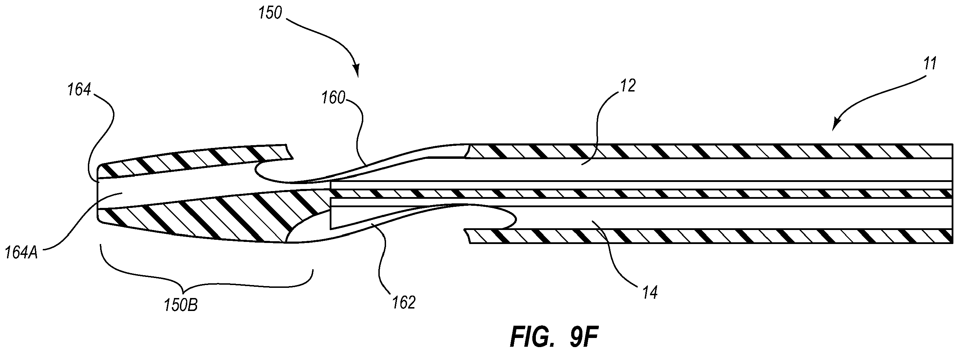

The distal tip region 150 defines a venous lateral opening 160 in fluid communication with the first lumen 12 and an arterial lateral opening 162 in fluid communication with the second lumen 14. A distal end opening 164 is also defined at a distal end of the nose portion 150B. The catheter assembly as configured in FIGS. 9A-9F is a dual lumen device in that it includes only two lumens 12 and 14 (FIG. 9E). As best seen in FIG. 9F, therefore, the distal end opening 164 does not communicate with a third lumen, but rather with a guidewire channel 164A defined by the nose portion 150B, which in turn communicates with the first lumen 12. In this way, a guidewire pathway is established through the catheter body 11 and distal tip region 150 to enable the catheter assembly to be inserted over a guidewire during initial placement and catheter exchange procedures.

FIG. 9E depicts a cross sectional view of the catheter body proximal of the distal tip region 150. As shown, top and bottom portions of an outer wall 186 of the catheter body 11 include thickened regions 186A, which provide added kink resistance to the catheter body.

By virtue of its communication with the first lumen 12, the guidewire channel 164A provides an added fluid outlet/inlet for the first lumen via the distal end opening 164, thus providing an additional fluid pathway that further reduces recirculation during operation of the catheter. This fluid communication also maintains the guidewire channel 164A patent via the flow of blood therethrough so as to prevent occlusion thereof. Further note that, though it is centrally located at the distal end of the nose portion 150B, the venous lateral opening 164 can be positioned such that it and the corresponding guidewire channel 164A are in longitudinal linear alignment with the first lumen 12. Further, the venous lateral opening and the corresponding guidewire channel can be configured as to be in communication with the second lumen or both the first and second lumens, if desired.

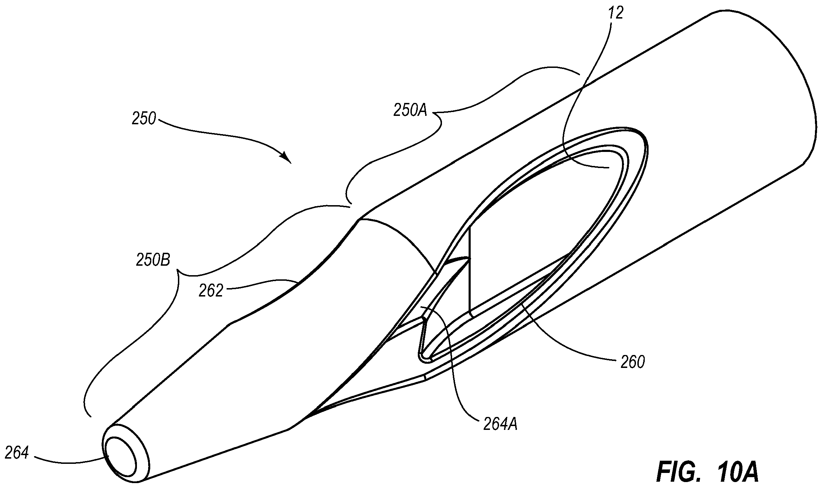

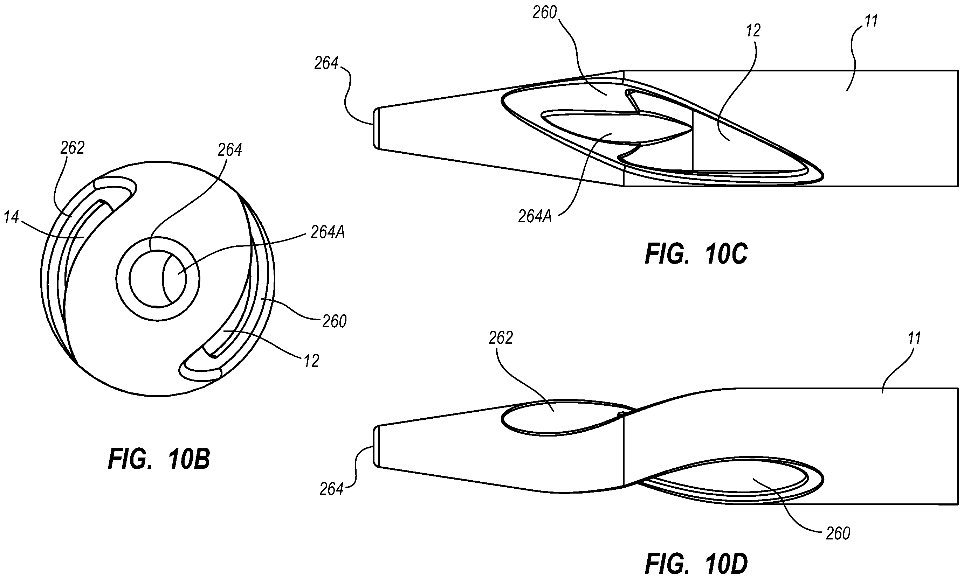

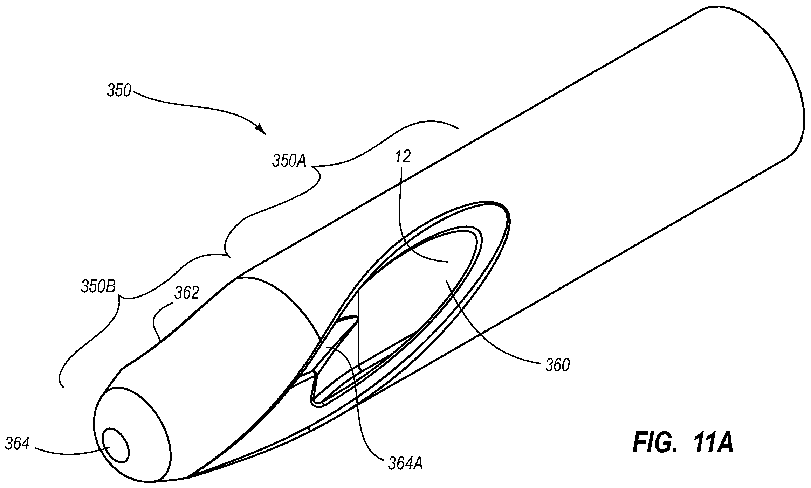

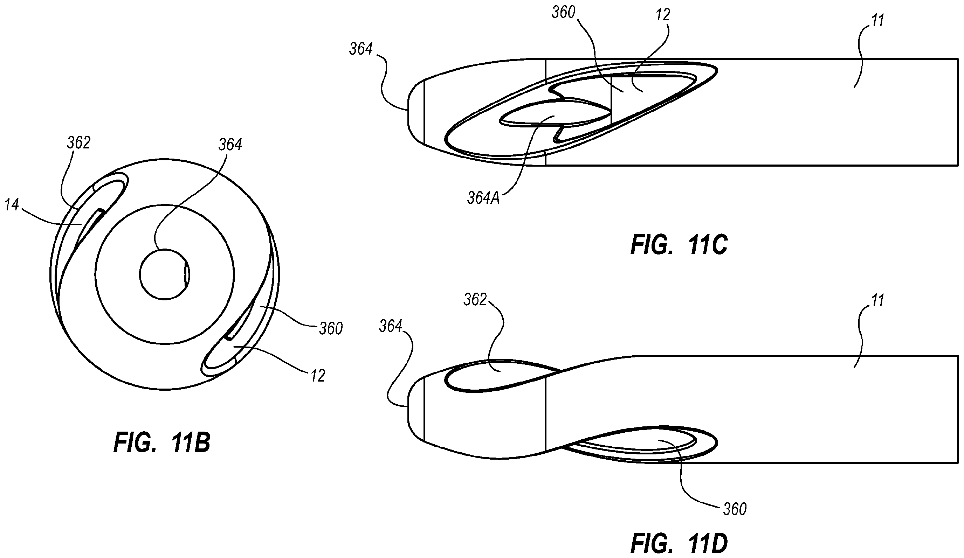

FIGS. 10A-10D and 11A-11D are further examples of a dual lumen catheter assembly configuration, in accordance with example embodiments thereof. The distal tip regions 250/350 each include a terminal catheter portion 250A/350A and a nose portion 250B/350B at which are defined a venous lateral opening 260/360, an arterial lateral opening 262/362, and a distal end opening 264/364. A guidewire channel 264A/364A is defined between the distal end opening 264/364 to the first lumen 12 so as to be in communication therewith. As can be seen in comparison, the lateral openings 260, 262 of FIGS. 10A-10D are differently shaped from corresponding lateral openings 360, 362 of FIGS. 11A-11D. Further, the nose portion 250B (FIG. 10A) is distally converging in a tapered configuration, whereas the nose portion 350B (FIG. 11A) distally converges in a rounded configuration to define a bullet-shape. Note also that the venous and arterial lateral openings of the dual lumen embodiments describe herein include distal-facing portions, as best seen in FIGS. 10B and 11B, offering characteristics similar to those outlined above in connection with the discussion relating to FIGS. 2-6.

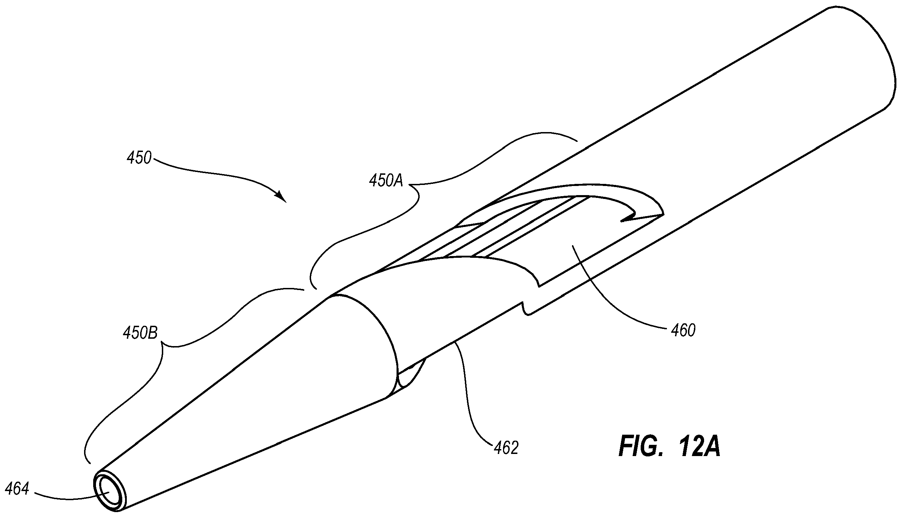

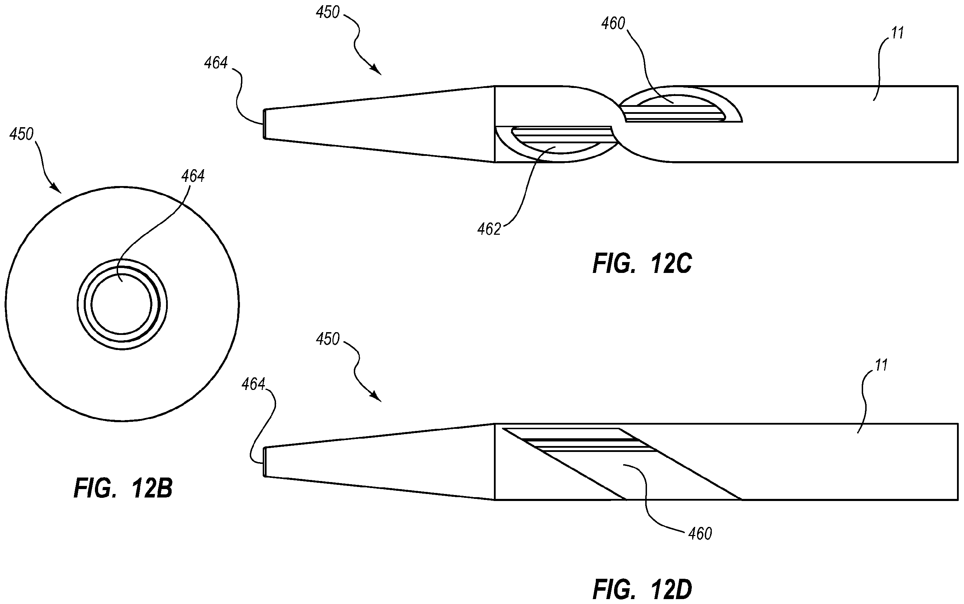

FIGS. 12A-20D depict possible configurations of a catheter assembly distal tip region including three lumens, according to additional example embodiments. As they share aspects with the embodiment described above in connection with FIGS. 2-7B, only selected aspects of the embodiments to follow will be discussed below.

FIGS. 12A-12D depicts a catheter assembly distal tip region 450, including a terminal catheter portion 450A and a nose portion 450B. The distal tip region 450 further includes a venous lateral opening 460 in fluid communication with the first lumen 12 and an arterial lateral opening 462 in fluid communication with the second lumen 14. A distal end opening 464 is also defined at a distal end of the nose portion 450B. In the present embodiment, the lateral openings 460 and 462 each define a trapezoidal perimeter when viewed from the perspective of FIG. 12D, and are symmetrically opposed from one another.

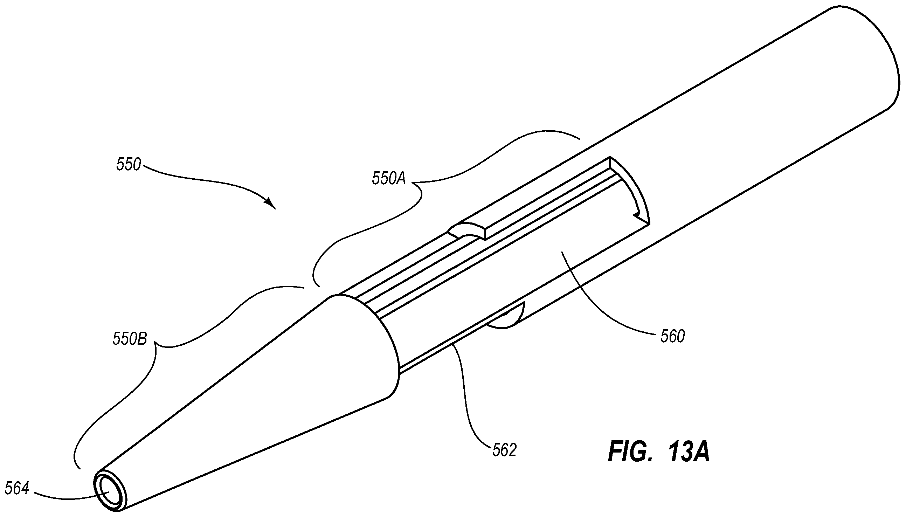

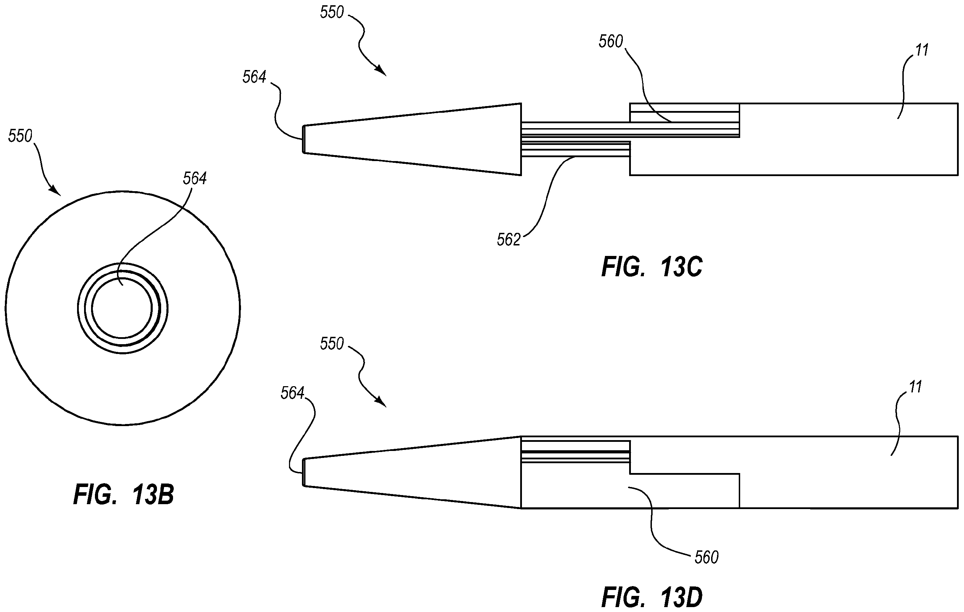

FIGS. 13A-13D depicts a catheter assembly distal tip region 550, including a terminal catheter portion 550A and a nose portion 550B. The distal tip region 550 further includes a venous lateral opening 560 in fluid communication with the first lumen 12 and an arterial lateral opening 562 in fluid communication with the second lumen 14. A distal end opening 564 is also defined at a distal end of the nose portion 550B. In the present embodiment, the lateral openings 460 and 462 each define a stepped perimeter when viewed from the perspective of FIG. 13D, and are symmetrically opposed from one another.

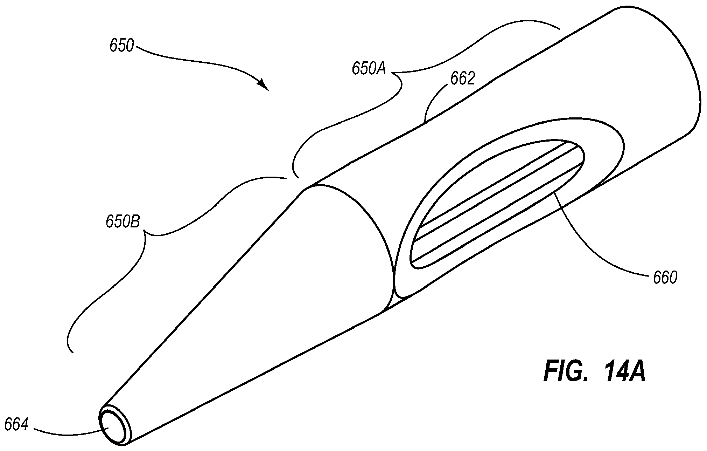

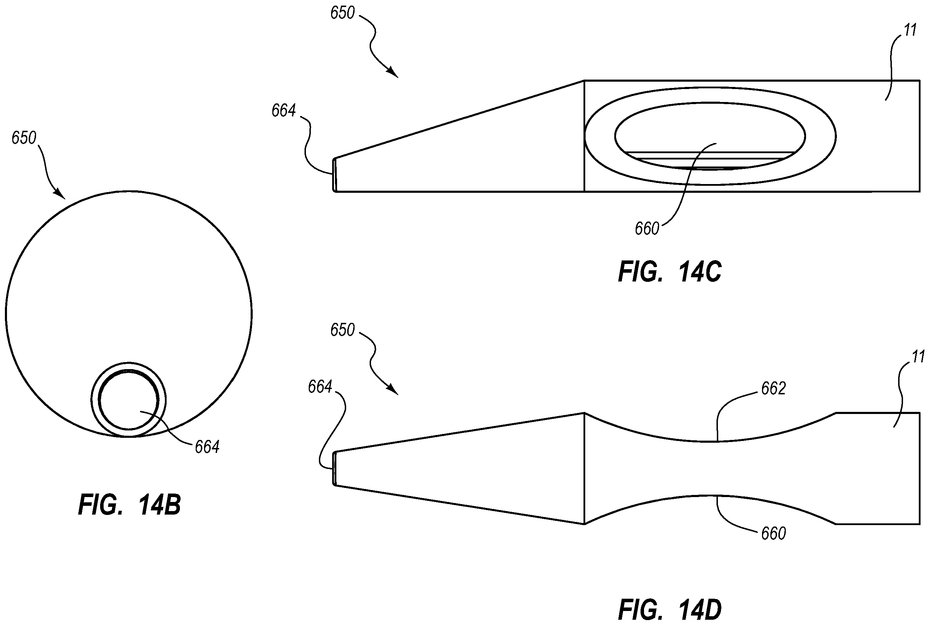

FIGS. 14A-14D depict a catheter assembly distal tip region 650, including a terminal catheter portion 650A and a nose portion 650B. The distal tip region 650 further includes a venous lateral opening 660 in fluid communication with the first lumen 12 and an arterial lateral opening 662 in fluid communication with the second lumen 14. A distal end opening 664 is also defined at a distal end of the nose portion 650B and is axially offset from a central axis of the catheter body 11. In the present embodiment, the lateral openings 660 and 662 each define an oval perimeter when viewed from the perspective of FIG. 12C, and are symmetrically opposed from one another, as best seen in FIG. 14D.

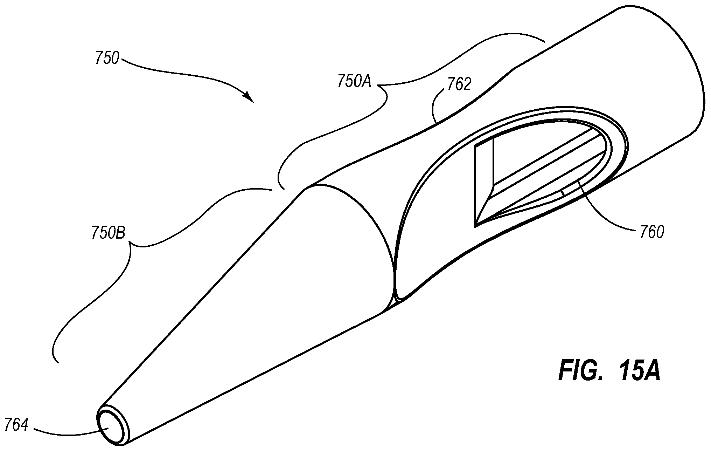

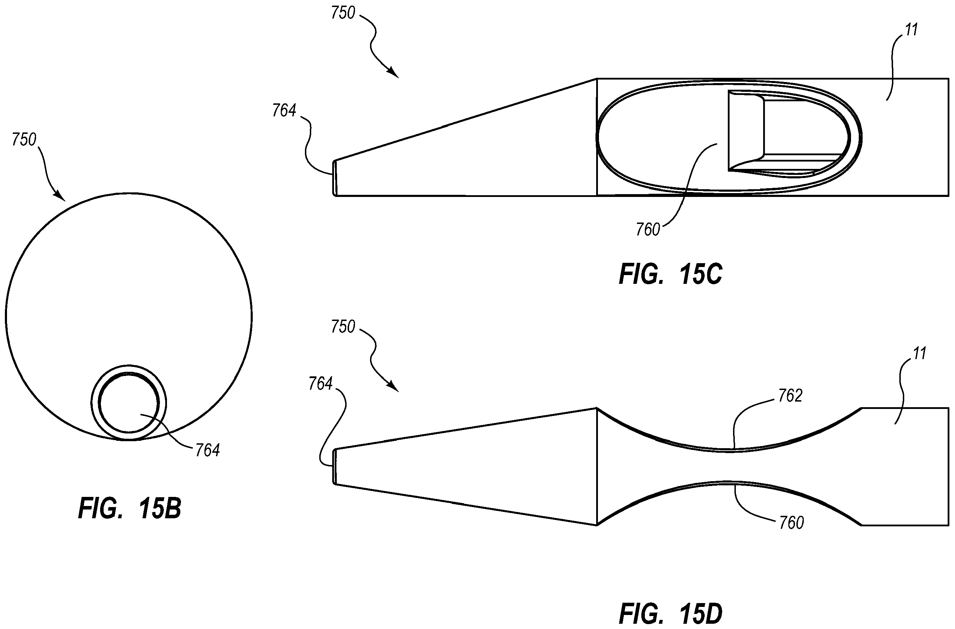

FIGS. 15A-15D depict a catheter assembly distal tip region 750, including a terminal catheter portion 750A and a nose portion 750B. The distal tip region 750 further includes a venous lateral opening 760 in fluid communication with the first lumen 12 and an arterial lateral opening 762 in fluid communication with the second lumen 14. A distal end opening 764 is also defined at a distal end of the nose portion 750B and is axially offset from a central axis of the catheter body 11. In the present embodiment, the lateral openings 760 and 762 each define an oval perimeter when viewed from the perspective of FIG. 15C, and are symmetrically opposed from one another, as best seen in FIG. 15D.

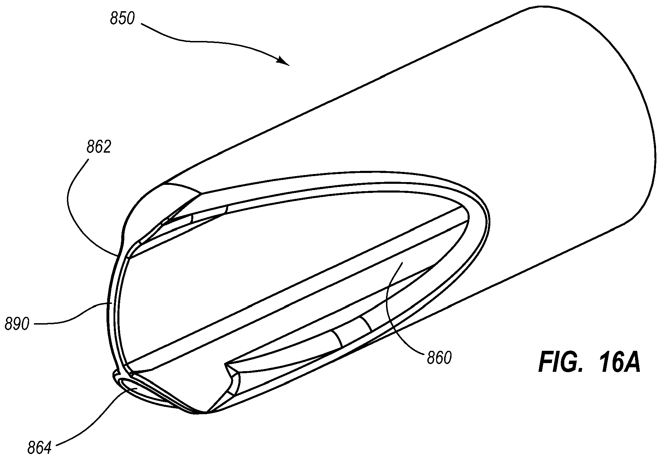

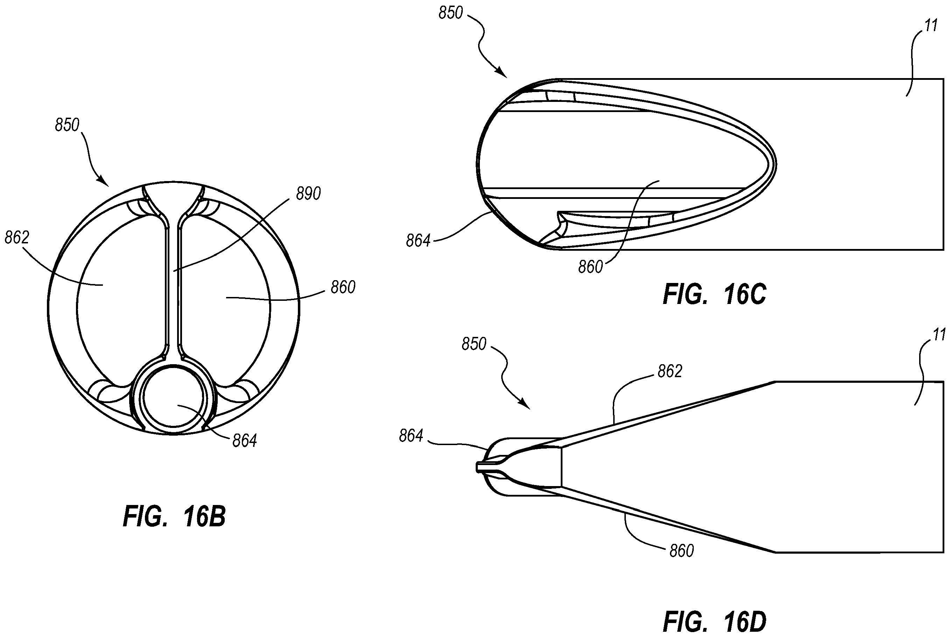

FIGS. 16A-16D depict a catheter assembly distal tip region 850, including a venous lateral opening 860 in fluid communication with the first lumen 12 and an arterial lateral opening 862 in fluid communication with the second lumen 14. A distal end opening 864 is also defined at a distal end of the distal tip region 850 and is axially offset from a central axis of the catheter body 11. In the present embodiment, the lateral openings 860 and 862 are separated by a septum 890, and each defines a partial oval perimeter when viewed from the perspective of FIG. 16C, and are symmetrically opposed from one another, as best seen in FIG. 16D.

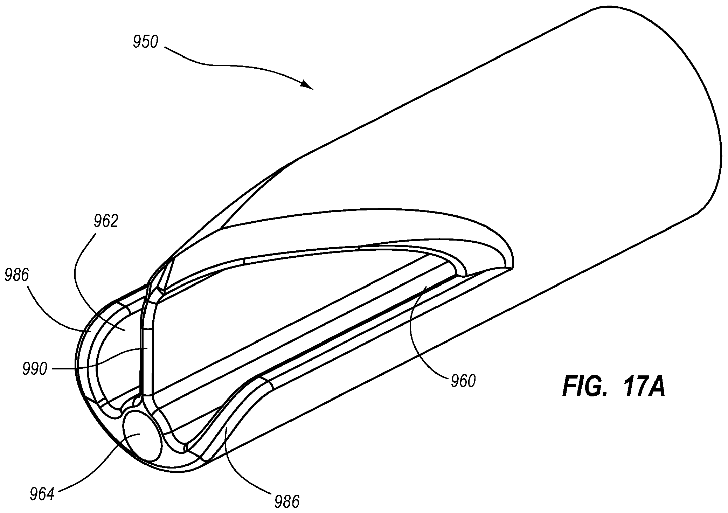

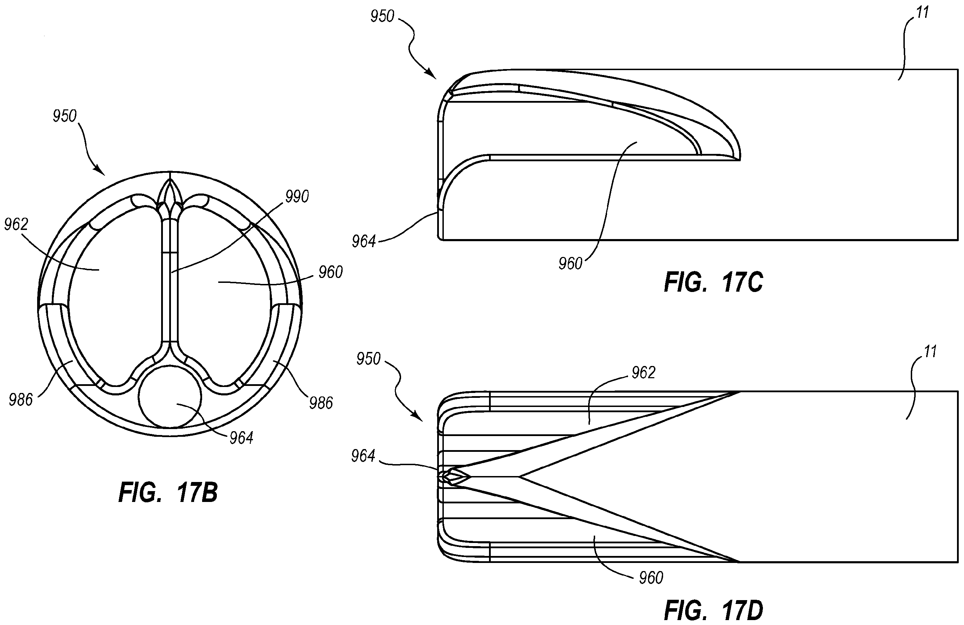

FIGS. 17A-17D depict a catheter assembly distal tip region 950, including a venous lateral opening 960 in fluid communication with the first lumen 12 and an arterial lateral opening 962 in fluid communication with the second lumen 14. A distal end opening 964 is also defined at a distal end of the distal tip region 850 and is axially offset from a central axis of the catheter body 11. In the present embodiment, the lateral openings 960 and 962 are separated by a septum 990, and each defines an acute angle-shaped perimeter together with a portion of an outer catheter body wall 986 when viewed from the perspective of FIG. 16C. As before, the lateral openings 960, 962 are symmetrically opposed from one another, as best seen in FIG. 17D.

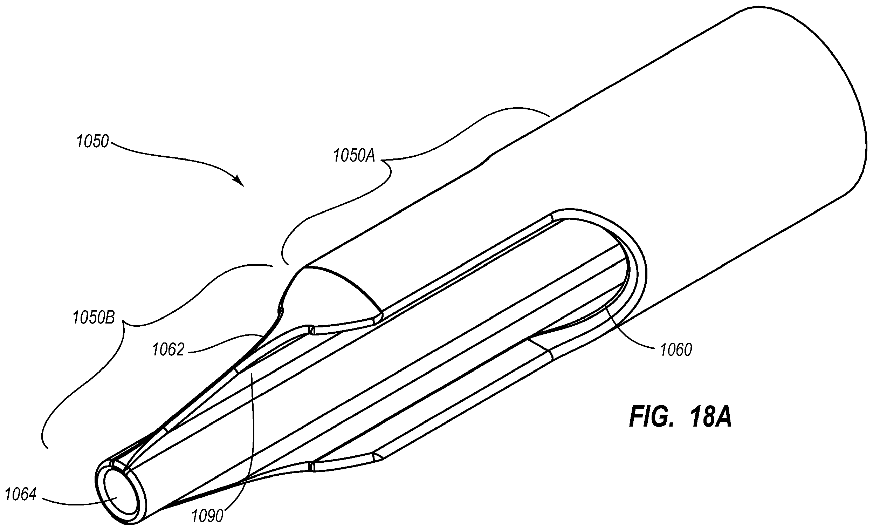

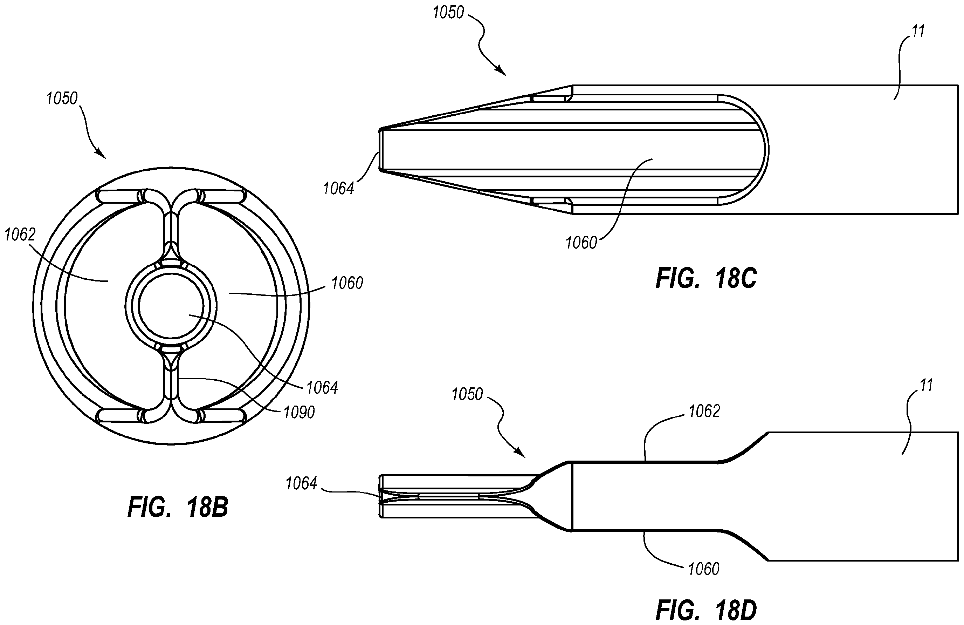

FIGS. 18A-18D depict a catheter assembly distal tip region 1050, including a terminal catheter portion 1050A and a nose portion 1050B. The distal tip region 1050 further includes a venous lateral opening 1060 in fluid communication with the first lumen 12 and an arterial lateral opening 1062 in fluid communication with the second lumen 14. A distal end opening 1064 is also defined at a distal end of the distal tip region nose portion 1050B and is centrally disposed with respect to a central axis of the catheter body 11. In the present embodiment, the lateral openings 1060 and 1062 are separated by a septum 1090, and each defines a partial oval perimeter when viewed from the perspective of FIG. 18C. As before, the lateral openings 1060, 1062 are symmetrically opposed from one another, as best seen in FIG. 18D.

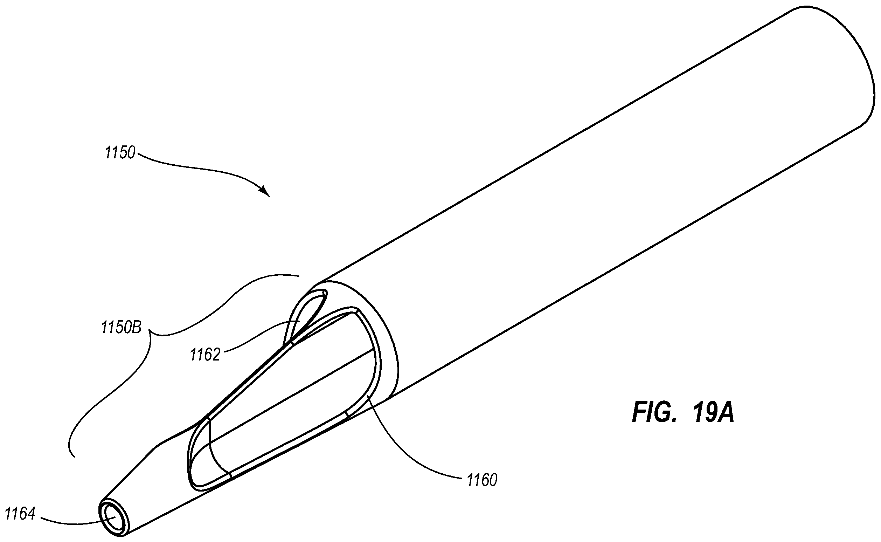

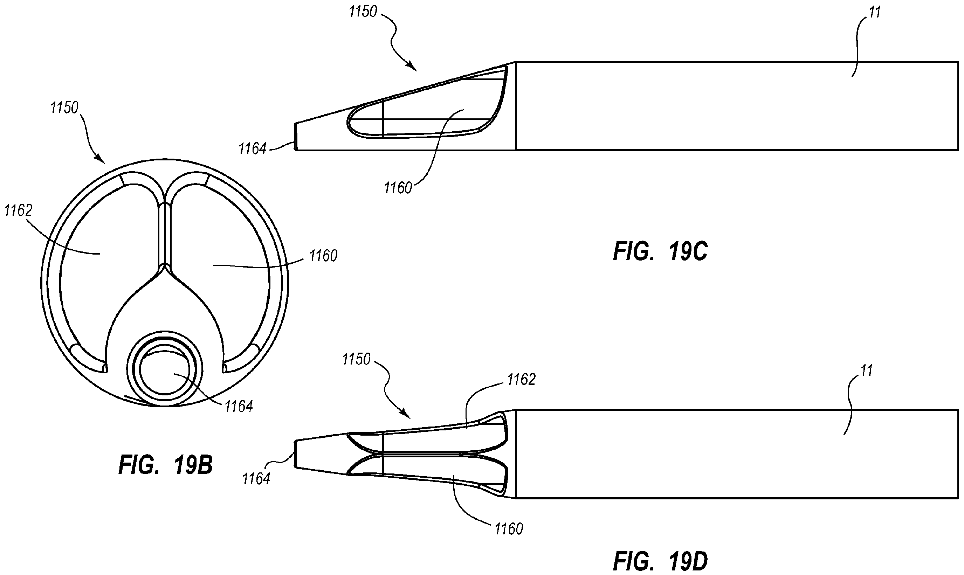

FIGS. 19A-19D depicts a catheter assembly distal tip region 1150, including a nose portion 1150B. The distal tip region 1150 further includes a venous lateral opening 1160 in fluid communication with the first lumen 12 and an arterial lateral opening 1162 in fluid communication with the second lumen 14. A distal end opening 1164 is also defined at a distal end of the distal tip region 1150 and is axially offset from a central axis of the catheter body 11. In the present embodiment, the lateral openings 1160 and 1162 each define a triangular perimeter when viewed from the perspective of FIG. 19D, and are symmetrically opposed from one another as best seen in FIG. 19D.

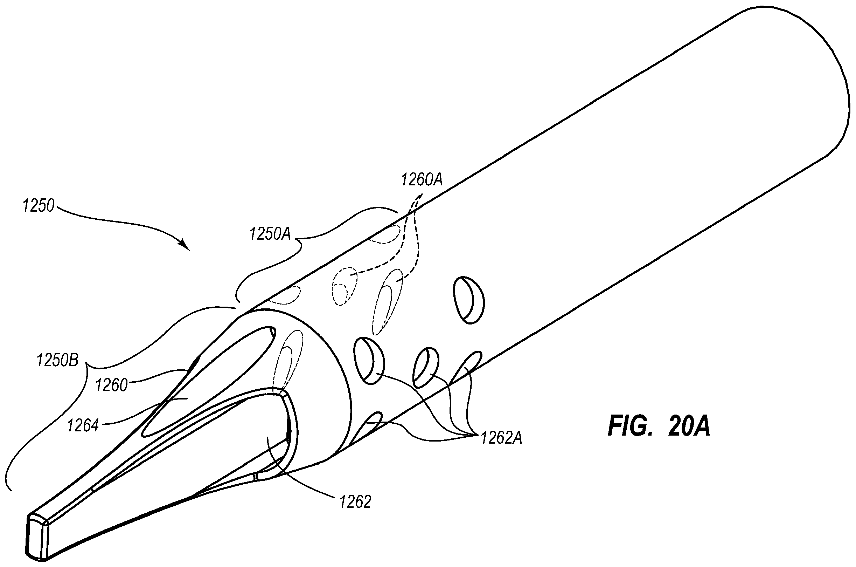

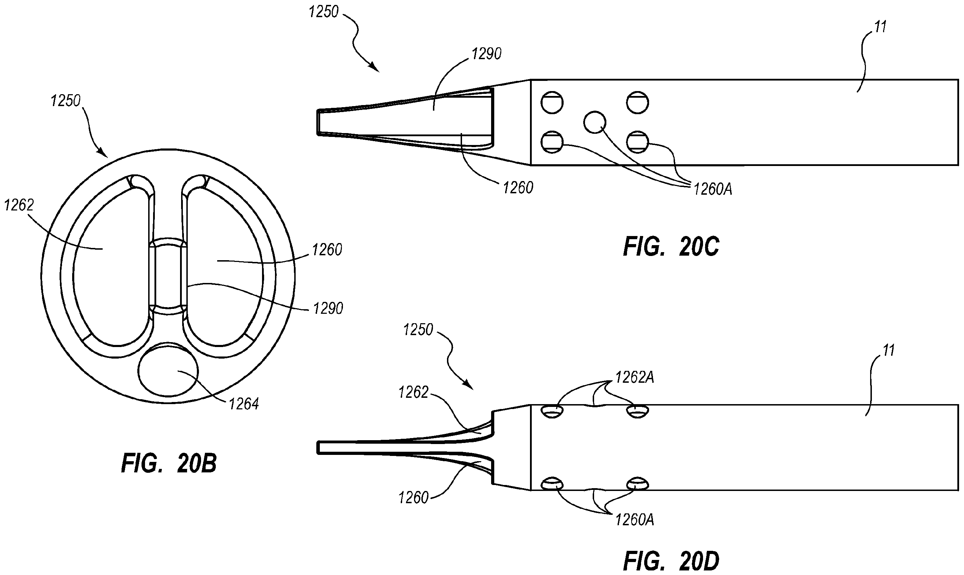

FIGS. 20A-20D depict a catheter assembly distal tip region 1250, including a terminal catheter portion 1250A and a nose portion 1250B. The distal tip region 1250 further includes a venous lateral opening 1260 in fluid communication with the first lumen 12 and an arterial lateral opening 1262 in fluid communication with the second lumen 14. A distal opening 1264 is also defined on the nose portion 1250B and is axially offset from a central axis of the catheter body 11. In the present embodiment, the lateral openings 1260 and 1262 are separated by a septum 1290, and each defines a frustoconical perimeter when viewed from the perspective of FIG. 20C. As before, the lateral openings 1260, 1262 are symmetrically opposed from one another, as best seen in FIG. 20D. In addition to the lateral openings 1260, 1262, the terminal catheter portion 1250A further includes a plurality of venous openings 1260A and a plurality of arterial openings 1262A. The openings 1260A, 1262A are relatively smaller than the lateral openings 1260, 1262, and are distributed about the perimeter of the catheter body so as to further reduce the possibility of vessel wall suck-up.

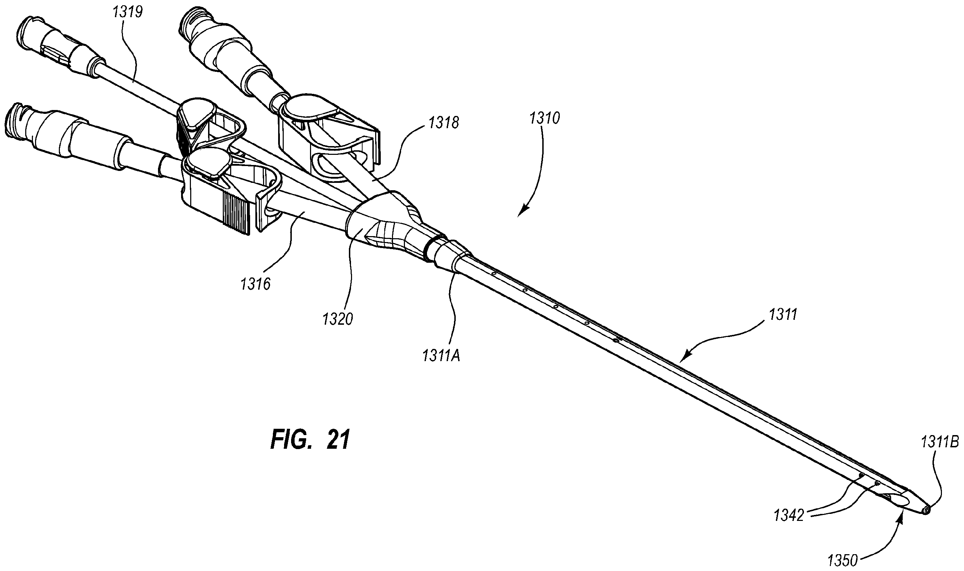

FIGS. 21-24 depict various details of a catheter assembly 1310 according to one embodiment. Note that the embodiments described below include various similarities to the embodiments described above; as such, only selected aspects will be discussed below.

As shown, the catheter assembly 1310 includes an elongate catheter tube, or catheter body 1311, which defines a plurality of lumens extending from a proximal end 1311A to a distal end 1311B. The proximal end 1311A of the catheter body 1311 is operably attached to a bifurcation 1320, which in turn is operably attached to extension legs, namely an arterial extension leg 1316, a venous extension leg 1318, and a power extension leg 1319 suitable for power injection of a fluid therethrough. The number of catheter body lumens, extension legs, and their respective configurations can vary from what is shown and described herein. For instance, though shown in FIG. 21 as straight, the arterial and venous extension legs 1316, 1318 can each be curved in a U-shaped configuration, in one embodiment. These and other modifications are contemplated. Note also that "bifurcation" is understood to include a hub that provide two or more fluid pathways.

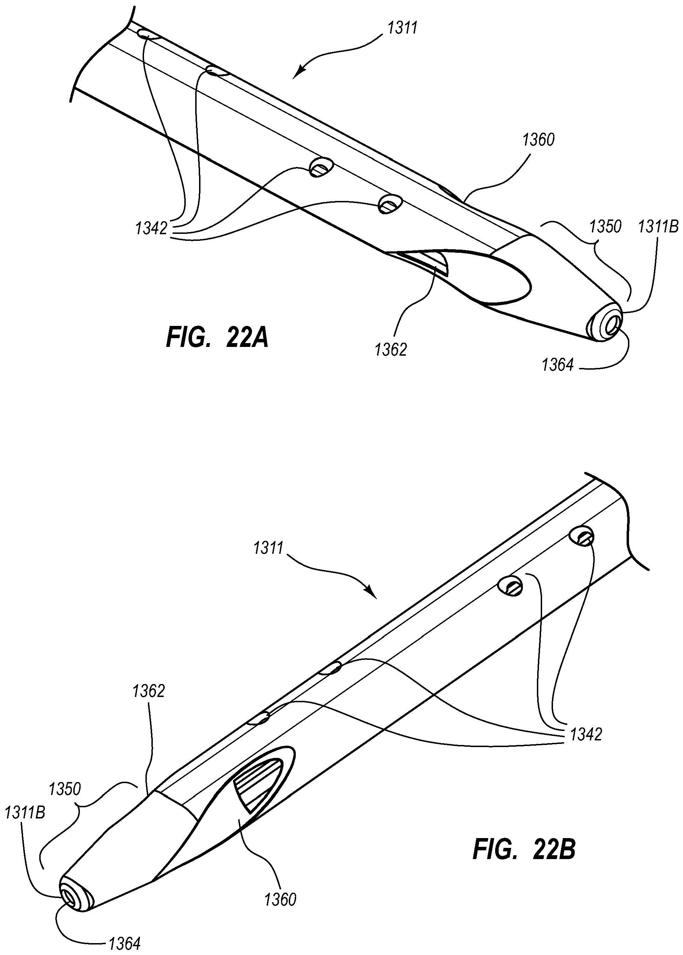

With continuing reference to FIG. 21, reference is made to FIGS. 22A and 22B, which depict distal portions of the catheter assembly 1310 and its elongate catheter body tube 1311, according to the present embodiment. As shown, the distal portion of the catheter body 1311 includes features similar to those shown in FIGS. 1-5 (discussed further above), including a tapered distal tip region 1350, in contrast to the cylindrically flattened oval-shaped outer surface of the more proximal portion of the catheter body, a venous lateral opening 1360, and an arterial lateral opening 1362. The arterial and venous and arterial lateral openings 1360 and 1362 are in fluid communication with respective arterial and venous lumens, which are referenced below and defined by the catheter body 1311. Each of the venous and arterial lateral openings 1360 and 1362 is defined by an angled skive cut so as to impart an angular direction component, with respect to the longitudinal axis of the catheter tube 1311, to fluid entering (via the arterial distal opening) or exiting (via the venous distal opening) the catheter tube, as before.

A third lumen distal end opening 1364 is included at the distal end of the distal tip region 1350 and is in fluid communication with a third lumen defined by the catheter body 1311, as discussed below. In addition, side holes 1342 are included in the catheter body 1311 proximal to the distal tip region 1350, which are in fluid communication with one of the arterial and venous lumens. Such side holes provide an alternate fluid path in addition to the venous and arterial lateral openings 1360, 1362. Note that the particular configuration of the various lateral and side hole openings can vary from what is shown and described herein.

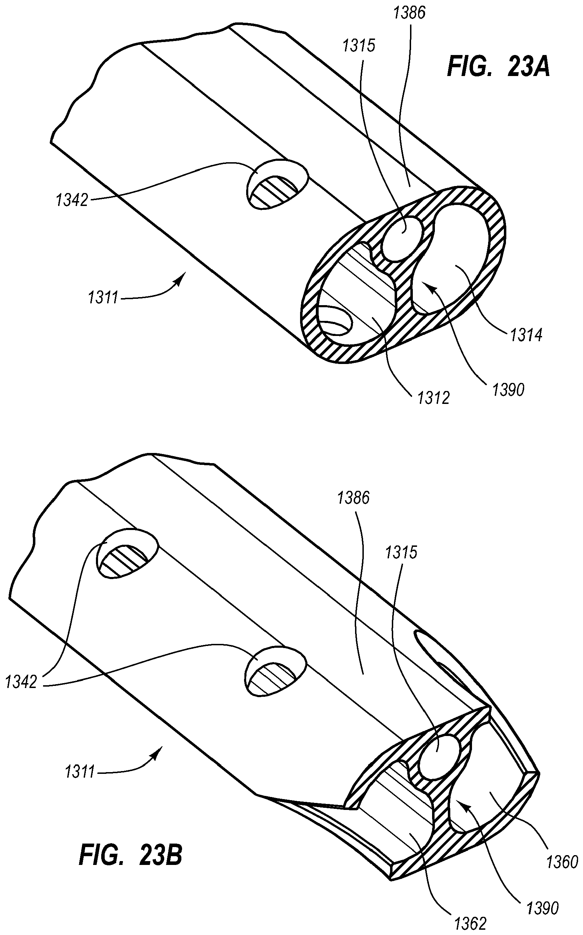

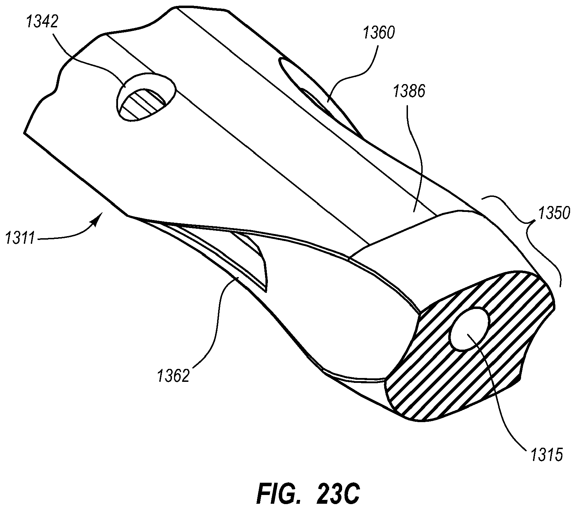

FIGS. 23A-23C depict the lumen configuration of the catheter body 1311 according to the present embodiment. As shown, an outer perimeter, or outer wall 1386 having a substantially flattened oval cross-sectional configuration defines the external portion of the catheter 1311. Indeed, the outer wall 1386 bounds a first, arterial lumen 1312, a second, venous lumen 1314, and a third lumen 1315, as mentioned above. A septum 1390 cooperates with the outer wall 1386 to define the particular shape configurations of the three lumens 1312, 1314, and 1315, which each substantially extend the longitudinal length of the catheter body 1311. FIG. 23B shows the manner in which the arterial lumen 1312 and venous lumen 1314 communicate with the arterial lateral opening 1362 and the venous lateral opening 1360, while FIG. 23C shows the manner in which the third lumen 1315 extends distally toward the distal end opening 1364 on the distal tip region 1350.

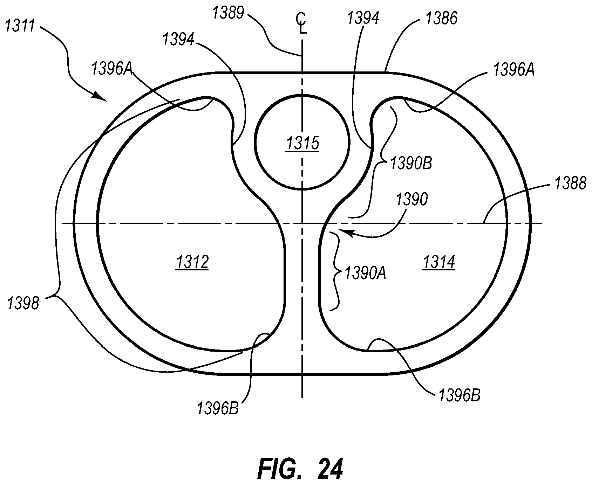

FIG. 24 depicts further details regarding the cross-sectional lumen configuration of the catheter body 1311, according to the present embodiment. As shown, the flattened oval outer wall 1386 and the septum 1390 of the catheter body 1311 define the arterial lumen 1312, the venous lumen 1314, and the third lumen 1315, as mentioned above. FIG. 24 shows that the third lumen 1315 has a cross-sectional shape that is substantially round and is configured in one embodiment to withstand fluid pressures typically associated with power injection, *e.g*., about 2.068 MPa (300 psi) in one example.

The cross-sectional configurations of the arterial and venous lumens 1312, 1314 are mirror projections of each other as taken across the center line ("CL") indicated at 1389 in FIG. 24. In particular, both the arterial and venous lumens 1312, 1314 cross-sectionally define a deformed kidney bean-shaped cross-sectional lumen profile, also referred to herein as a modified reniform shape. In greater detail, each of the arterial and venous lumens 1312, 1314 cross-sectionally defines a concavely-shaped portion, or concavity 1394, which contributes to the reniform lumen shape. The concavity 1394 for each lumen 1312, 1314 is disposed above a transverse axis 1388 of the catheter body 1311 as shown in and from the perspective of FIG. 24. Disposal of the concavity 1394 of each lumen 1312, 1314 above the transverse axis 1388, as opposed to the concavity being centered on the transverse axis results in a modified reniform configuration, though it is appreciated that the size and location of the concavity can vary from what is shown and described herein. Indeed, in one embodiment the concavity can be positioned so as to define a general reniform (un-deformed kidney bean) shape.

Each lumen 1312, 1314 further includes an arcuate portion, or major arc 1398, opposite the respective concavity 1394 that defines an outer portion of each lumen adjacent the outer wall 1386. The major arc 1398 of each lumen 1312, 1314 is bounded on either end by a top corner 1396A and a bottom corner 1396B. This configuration interposes the top corner 1396A between the major arc 1398 and the concavity 1394. The top and bottom corners 1396A and 1396B are substantially rounded to ensure a laminar flow of fluids through the arterial and venous lumens 1312, 1314, thus desirably preventing areas of fluid flow stagnation.

As shown in FIG. 24, the septum 1390 is included to separate the arterial lumen, 1312, the venous lumen 1314, and the third lumen 1315. Centered on the center line 1389, the septum 1390 includes a unified portion 1390A that generally extends downward from the transverse axis 1388 (from the perspective shown in FIG. 24) and a bifurcated portion 1390B that generally extends upward from the transverse axis. Particularly, the septum 1390 helps define the aformentioned shapes of the lumens. For example, the unified portion 1390A of the septum 1390 generally defines an hourglass-like cross-sectional shape to help define the rounded bottom corners 1396B and the inner portions of both the arterial lumen 1312 and venous lumen 1314, while the bifurcated portion 1390B of the septum cooperates with the outer wall 1386 to define the cross-sectional shape of the third lumen 1315 and the concavities 1394 of the arterial and venous lumens. Note also that the general hourglass configuration of the septum 1390 adds structural strength to the septum.

The cross-sectional configuration shown in FIG. 24 in the present embodiment extends from the proximal end 1311A of the catheter body 1311 distally to the arterial and venous lateral openings 1362, 1360, though this can be modified in other embodiments. It is noted that the various cross-sectional features of the catheter body 1311 described immediately above can vary in size, shape, and position from what is shown and described herein.

According to one embodiment, the various features described above include the following cross-sectional dimensions: the perimeter of the outer wall 1386 includes a width of about 4.953 mm (0.195 inch) and a height of about 3.251 mm (0.128 inch); the diameter of the third lumen is about 1.016 mm (0.040 inch); the thickness of the unified portion 1390A of the septum 1390 is about 0.381 mm (0.015 inch); the thickness of each branch of the bifurcated portion 1390B of the septum 1390 at the midpoint of the respective concavity 1394 is about 0.254 mm (0.010 inch); the distance between the outer surface of the outer wall and the nearest point of the third lumen is about 0.254 mm (0.010 inch); the thickness of the outer wall at about the midpoint of the major arc 1398 is about 0.381 mm (0.015 inch); the radius of each concavity of the identical arterial and venous lumens 1312, 1314 as measured from a center point of the third lumen is about 0.76 mm (0.030 inch); the radius of each top corner 1396A is about 0.305 mm (0.012 inch); the radius of each bottom corner 1396B is about 0.508 mm (0.020 inch); the radius of each major arc is about 1.320 mm (0.052 inch); the radius at the end of the concavity opposite the top corner (at about the transverse axis 1388) is about 0.762 mm (0.030 inch); and the distance between the outer surface of the outer wall and the nearest point of arterial or venous lumen proximate the bottom corner thereof is about 0.254 mm (0.010 inch). Note that the lumen configuration of the present embodiment enables fluid flow therethrough equal to a known 13 French-sized catheter while occupying the size of only a 12 French catheter. Of course, the size of the catheter body and its respective lumens can be scaled as needed/desired.

The catheter body 1311 in one embodiment includes a suitable thermoplastic such as polyurethane, for instance. In some embodiments, polyurethane thermoplastics sold under the marks TECOFLEX®, CARBOTHANE®, CHRONOFLEX®, and QUADRIFLEX® can be used to form the catheter tube. Note that other suitable, biocompatible materials can also be used. In one embodiment, the catheter tube 12 includes a polyurethane with a 60D Shore hardness, which assists in preventing kinking, enabling power injection therethrough, and improving insertability into the body of a patient in a acute dialysis scenario, for instance. In other non-limiting embodiments, the hardness of the catheter tube can vary from about 55D to about 65D. Desired characteristics for the material from which the catheter body is formed in one embodiment include thermosensitivity such that the material softens after insertion into the patient body, and suitable polymer strength to withstand power injection pressures to which the catheter assembly may be subjected.

In one embodiment, the atraumatic tip of the distal tip region 1350 includes a polyurethane with an 85A Shore hardness. In one non-limiting example, the atraumatic tip can range from 85A to 75A Shore hardness. In one embodiment, the material of the catheter body 1311 and atraumatic tip can include a radiopaque material, such as barium or tungsten, to enable visibility of the catheter assembly under x-ray imaging.

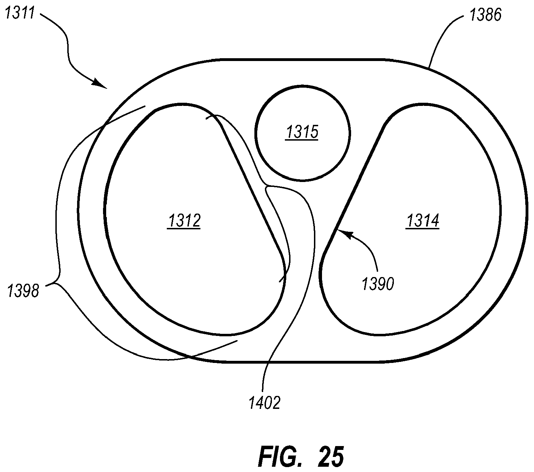

FIG. 25 depicts the catheter body 1311 according to another embodiment, wherein the arterial and venous lumens 1312, 1314 include a differing cross-sectional configuration from that shown in FIG. 24. As shown, the substantially identical arterial and venous lumens 1312, 1314 each cross-sectionally define the major arc 1398 and opposite thereto a flattened side 1402, defined by the septum 1390.

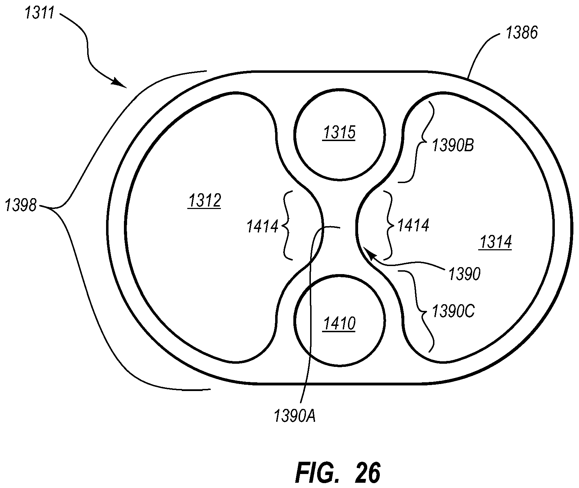

FIG. 26 depicts the catheter body 1311 according to another embodiment, wherein the arterial and venous lumens 1312, 1314 include a differing cross-sectional configuration from that shown in FIG. 24. As shown, a fourth lumen 1410, substantially round in cross-sectional shape, is included. Further, the substantially identical arterial and venous lumens 1312, 1314 each cross-sectionally define the major arc 1398 and opposite thereto a convex portion 1414, defined by the septum 1390. In particular, the septum 1390 includes a centrally disposed unified portion 1390A and a first and second bifurcated portion 1390B, 1390C that are disposed on either side of the unified portion and largely define the third lumen 1315 and fourth lumen 1410.

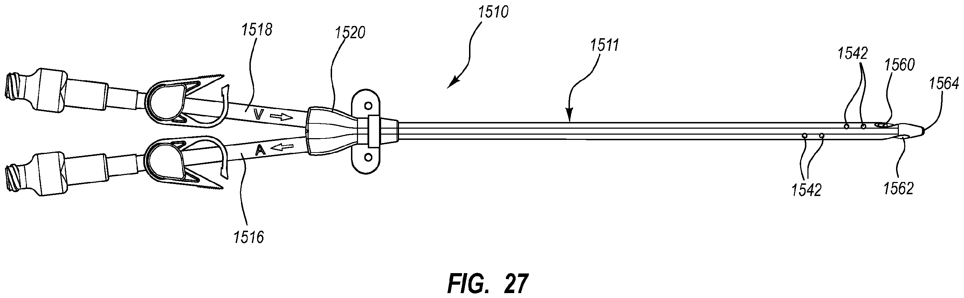

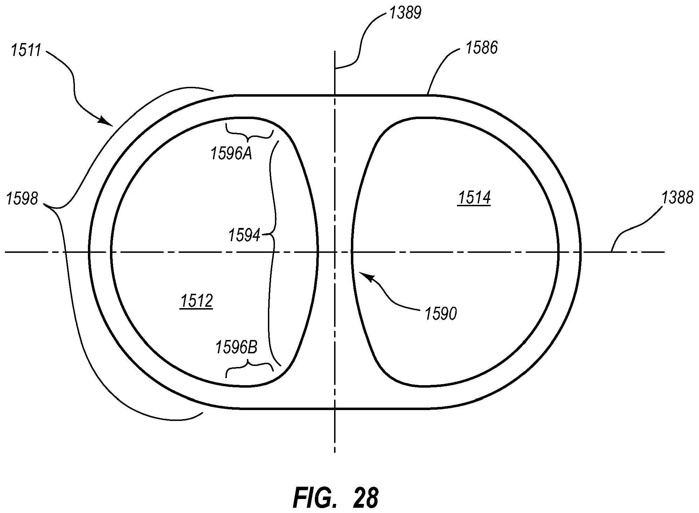

FIGS. 27 and 28 depict various details of a catheter assembly 1510 according to one embodiment. Note that the embodiments described below include various similarities to the embodiments described above; as such, only selected aspects will be discussed below.

As shown, the catheter assembly 1510 includes an elongate catheter tube, or catheter body 1511, which defines a plurality of lumens extending from a proximal end to a distal end thereof. The proximal end of the catheter body 1511 is operably attached to a bifurcation 1520, which in turn is operably attached to extension legs, namely an arterial extension leg 1516 and a venous extension leg 1518. The number of catheter body lumens, extension legs, and their respective configurations can vary from what is shown and described herein. For instance, though shown in FIG. 27 as straight, the arterial and venous extension legs 1316, 1318 can each be curved in a U-shaped configuration, in one embodiment. These and other modifications are contemplated.

The distal portion of the catheter body 1511 includes features similar to those shown in FIGS. 1-5 (discussed further above), including a tapered distal tip region in contrast to the cylindrically flattened oval-shaped outer surface of the more proximal portion of the catheter body, a venous lateral opening 1560, and an arterial lateral opening 1562. The venous and arterial lateral openings 1560 and 1562 are in fluid communication with respective venous and arterial lumens, which are referenced below and defined by the catheter body 1511. Each of the venous and arterial lateral openings 1560 and 1562 is defined by an angled skive cut so as to impart an angular direction component, with respect to the longitudinal axis of the catheter tube 1511, to fluid entering (via the arterial distal opening) or exiting (via the venous distal opening) the catheter tube, as before.

A distal end opening 1564 is included at the distal end of the distal tip region and is in fluid communication with the venous lumen, described below, though the distal end opening could be in communication with the arterial lumen in another embodiment. In addition, side holes 1542 are included in the catheter body 1511 proximal to the distal tip region, which are in fluid communication with one of the arterial and venous lumens. Such side holes provide an alternate fluid path in addition to the venous and arterial lateral openings 1560, 1562. Note that the particular configuration of the various lateral and side hole openings can vary from what is shown and described herein.

FIG. 28 depicts further details regarding the cross-sectional lumen configuration of the catheter body 1511, according to the present embodiment. As shown, an outer perimeter, or outer wall 1586 having a substantially flattened oval cross-sectional configuration defines the external portion of the catheter 1511. Indeed, the outer wall 1586 bounds a first, arterial lumen 1512 and a second, venous lumen 1514, as mentioned above. A septum 1590 cooperates with the outer wall 1586 to define the particular shape configurations of the two lumens 1512 and 1514, which each substantially extend the longitudinal length of the catheter body 1511. As discussed, the arterial lumen 1512 and the venous lumen 1514 communicate with the arterial lateral opening 1562 and the venous lateral opening 1560, respectively.

FIG. 28 depicts further details regarding the cross-sectional lumen configuration of the catheter body 1511, according to the present embodiment. As shown, the flattened oval outer wall 1586 and the hourglass-shaped septum 1590 of the catheter body 1511 define the arterial lumen 1512 and the venous lumen 1514, as mentioned above. The cross-sectional configurations of the arterial and venous lumens 1512, 1514 are mirror projections of each other as taken across the center line ("CL") indicated at 1389 in FIG. 28. In particular, both the arterial and venous lumens 1512, 1514 cross-sectionally define a modified ellipse cross-sectional lumen profile. In greater detail, each of the arterial and venous lumens 1512, 1514 cross-sectionally defines a first, minor arc 1594 adjacent and defined by the hourglass-shaped septum 1590, bounded by two corners: a top corner 1596A and a bottom corner 1596B. A second, major arc 1598 extends from each of the corners 1596A, 1596B on a side opposite the septum 1590 and adjacent the outer wall 1586 to define the rest of each lumen 1512, 1514. This configuration interposes both the top corner 1596A and the bottom corner 1596B between the major arc 1598 and the minor arc 1594. The top and bottom corners 1596A and 1596B are substantially rounded to ensure a laminar flow of fluids through the arterial and venous lumens 1512, 1514, thus desirably preventing areas of fluid flow stagnation.

As shown in FIG. 28, the septum 1590 separates the arterial lumen 1512 and the venous lumen 1514. Centered on the center line 1389, the septum 1590 defines an hourgalss cross-sectional shape equally distributed about the transverse axis 1388 and helps define the aformentioned shapes of the lumens. Note that the general hourglass configuration of the septum 1590 adds structural strength to the septum.

The cross-sectional configuration shown in FIG. 28 in the present embodiment extends from the proximal end of the catheter body 1511 distally to the arterial and venous lateral openings 1562, 1560, though this can be modified in other embodiments. It is noted that the various cross-sectional features of the catheter body 1511 described immediately above can vary in size, shape, and position from what is shown and described herein.

According to one embodiment, the various features described above include the following cross-sectional dimensions: the perimeter of the outer wall 1386 includes a width of about 4.394 mm (0.173 inch) and a height of about 2,921 mm (0.115 mm); the thickness of the septum 1390 at the transverse axis 1388 is about 0.381 mm (0.015 inch); the thickness of outer wall along the major arc 1598 is about 0.254 mm (0.010 inch); the radius of the minor arc 1594 is about 2.54 mm (0.100 inch); the radius of the major arc 1598 is about .050 inch; the width of each lumen 1512, 1514 at the transverse axis 1388 is about 1.828 mm (0.072 inch); and the radius of each corner 1596A, 1596B is about 0.406 mm (0.016 inch). Note that the above dimensions pertain to a catheter assembly 1510 having an 11 French size; of course, the size of the catheter body and its respective lumens can be scaled as needed/desired. The catheter body 1511 and its atraumatic tip can include suitable materials as have been described further above.

## Claims

1. A catheter assembly (10), comprising:
an elongate catheter tube (11) defining first (12), second (14), and third (15) lumens, wherein:
the first (12) and second (14) lumens include a modified reniform cross-sectional shape;
the third lumen (15) includes a substantially circular cross sectional shape; and
the catheter tube (11) includes:
a first lateral opening (60) in fluid communication with the first lumen (12), the first lateral opening defined by an angled cross cut; and
a second lateral opening (62) in fluid communication with the second lumen (14), the second lateral opening defined by an angled cross cut.

2. The catheter assembly as defined in claim 1, wherein the first and second lumens (12, 14) are mirror images of one another about a center line of the catheter tube.

3. The catheter assembly as defined in claim 1, wherein a cross section of the outer surface of the catheter tube (11) defines a flattened oval shape.

4. The catheter assembly as defined in claim 1, wherein the third lumen (15) is configured to withstand pressures associated with power injection of a fluid therethrough.

5. The catheter assembly as defined in claim 4, wherein the catheter tube (11) further includes a distal end opening in fluid communication with the third lumen (15).

6. The catheter assembly as defined in claim 5, wherein the catheter tube (11) includes a tapered distal region, the distal end opening disposed at a distal tip (50) of the tapered distal region.

7. The catheter assembly as defined in claim 6, wherein the first and second lateral openings (60, 62) are positioned in an un-staggered position on a distal portion of the catheter tube.

8. The catheter assembly as defined in claim 7, wherein a portion of the first lateral opening (60) and the second lateral opening (62) is disposed on the tapered distal region.

9. The catheter assembly as defined in claim 1, wherein a septum (90) separates the first lumen (12), the second lumen (14), and the third lumen (15), the septum including an hourglass-shaped portion.

10. The catheter assembly as defined in claim 1, wherein each of the first and second lumens (12, 14) cross-sectionally includes a concavity (1394), the concavity disposed adjacent the third lumen (15).

11. The catheter assembly as defined in claim 10, wherein each of the first and second lumens (12, 14) cross-sectionally includes first and second corners (1396A, 1396B), the concavity interposed in an offset configuration between the first and second corners.

## Patentansprüche

1. Katheteranordnung (10), umfassend:
einen länglichen Katheterschlauch (11), der ein erstes (12), zweites (14) und drittes (15) Lumen definiert, wobei:
das erste (12) und zweite (14) Lumen eine modifizierte reniforme Querschnittsform beinhalten;
das dritte Lumen (15) eine im Wesentlichen kreisförmige Querschnittsform beinhaltet;
und
der Katheterschlauch (11) Folgendes beinhaltet:
eine erste seitliche Öffnung (60) in Fluidverbindung mit dem ersten Lumen (12), wobei die erste seitliche Öffnung durch einen abgewinkelten Querschnitt definiert ist; und
eine zweite seitliche Öffnung (62) in Fluidverbindung mit dem zweiten Lumen (14), wobei die zweite seitliche Öffnung durch einen abgewinkelten Querschnitt definiert ist.

2. Katheteranordnung nach Anspruch 1, wobei das erste und zweite Lumen (12, 14) Spiegelbilder voneinander um eine Mittellinie des Katheterschlauchs sind.

3. Katheteranordnung nach Anspruch 1, wobei ein Querschnitt der Außenfläche des Katheterschlauchs (11) eine abgeflachte ovale Form definiert.

4. Katheteranordnung nach Anspruch 1, wobei das dritte Lumen (15) konfiguriert ist, um Drücken standzuhalten, die mit der Energieeinspeisung eines Fluids durch sie verbunden sind.

5. Katheteranordnung nach Anspruch 4, wobei der Katheterschlauch (11) weiter eine distale Endöffnung in Fluidverbindung mit dem dritten Lumen (15) beinhaltet.

6. Katheteranordnung nach Anspruch 5, wobei der Katheterschlauch (11) einen verjüngten distalen Bereich beinhaltet, wobei die distale Endöffnung an einer distalen Spitze (50) des verjüngten distalen Bereichs angeordnet ist.

7. Katheteranordnung nach Anspruch 6, wobei die erste und zweite seitliche Öffnung (60, 62) in einer nicht gestaffelten Position auf einem distalen Abschnitt des Katheterschlauchs positioniert sind.

8. Katheteranordnung nach Anspruch 7, wobei ein Abschnitt der ersten seitlichen Öffnung (60) und der zweiten seitlichen Öffnung (62) auf dem konischen distalen Bereich angeordnet ist.

9. Katheteranordnung nach Anspruch 1, wobei ein Septum (90) das erste Lumen (12), das zweite Lumen (14) und das dritte Lumen (15) trennt, wobei das Septum einen sanduhrförmigen Abschnitt beinhaltet.

10. Katheteranordnung nach Anspruch 1, wobei jedes der ersten und zweiten Lumen (12, 14) im Querschnitt eine Konkavität (1394) beinhaltet, wobei die Konkavität benachbart zum dritten Lumen (15) angeordnet ist.

11. Katheteranordnung nach Anspruch 10, wobei jedes der ersten und zweiten Lumen (12, 14) im Querschnitt erste und zweite Ecken (1396A, 1396B) beinhaltet, wobei die Konkavität in einer versetzten Konfiguration zwischen den ersten und zweiten Ecken eingefügt ist.

## Revendications

1. Ensemble cathéter (10), comprenant :
un tube de cathéter allongé (11) définissant des première (12), deuxième (14) et troisième (15) lumières, dans lequel :
les première (12) et deuxième (14) lumières comprennent une forme de section transversale réniforme modifiée ;
la troisième lumière (15) comprend une forme de section transversale sensiblement circulaire ; et
le tube de cathéter (11) comprend :
une première ouverture latérale (60) en communication fluidique avec la première lumière (12), la première ouverture latérale étant définie par une coupe transversale angulaire ; et
une seconde ouverture latérale (62) en communication fluidique avec la deuxième lumière (14), la seconde ouverture latérale étant définie par une coupe transversale angulaire.

2. Ensemble cathéter tel que défini dans la revendication 1, dans lequel les première et deuxième lumières (12, 14) sont des images miroir l'une de l'autre autour d'une ligne centrale du tube de cathéter.

3. Ensemble cathéter tel que défini dans la revendication 1, dans lequel une section transversale de la surface extérieure du tube de cathéter (11) définit une forme ovale aplatie.

4. Ensemble cathéter tel que défini dans la revendication 1, dans lequel la troisième lumière (15) est configurée pour supporter des pressions associées à une injection en puissance d'un fluide à travers celle-ci.

5. Ensemble cathéter tel que défini dans la revendication 4, dans lequel le tube de cathéter (11) comprend en outre une extrémité distale s'ouvrant en communication fluidique avec la troisième lumière (15).

6. Ensemble cathéter tel que défini dans la revendication 5, dans lequel le tube de cathéter (11) comprend une région distale effilée, l'ouverture d'extrémité distale étant disposée au niveau d'une pointe distale (50) de la région distale effilée.

7. Ensemble cathéter tel que défini dans la revendication 6, dans lequel les première et seconde ouvertures latérales (60, 62) sont positionnées dans une position non-décalée sur une partie distale du tube de cathéter.

8. Ensemble cathéter tel que défini dans la revendication 7, dans lequel une partie de la première ouverture latérale (60) et de la seconde ouverture latérale (62) est disposée sur la région distale effilée.

9. Ensemble cathéter tel que défini dans la revendication 1, dans lequel un septum (90) sépare la première lumière (12), la deuxième lumière (14) et la troisième lumière (15), le septum comprenant une partie en forme de sablier.

10. Ensemble cathéter tel que défini dans la revendication 1, dans lequel chacune des première et seconde lumières (12, 14) inclut en section transversale une concavité (1394), la concavité étant disposée adjacente à la troisième lumière (15).

11. Ensemble cathéter tel que défini dans la revendication 10, dans lequel chacune des première et deuxième lumières (12, 14) comprend en section transversale des premier et second coins (1396A, 1396B), la concavité étant interposée dans une configuration décalée entre les premiers et seconds coins.
